# EUROPEAN PATENT APPLICATION

(11) **EP 4 768 814 A1**
(43) Date of publication of application: **01.07.2026**
(21) Application number: 24877410.1
(22) Date of filing: 06.09.2024
(51) Int. Cl.: F24F 11/66, F24F 11/70, F24F 11/56, A61B 5/00, F24F 120/10, F24F 130/30

(54) **AIR CONDITIONER FOR PROVIDING SLEEP MODE, AND CONTROL METHOD THEREFOR**

(30) Priority: 11.10.2023 KR 20230135407
(71) Applicant: Samsung Electronics Co., Ltd., Suwon-si, Gyeonggi-do 16677 (KR)
(72) Inventor: LEE, Jongmoon, Suwon-si, Gyeonggi-do 16677 (KR); KIM, Donghyun, Suwon-si, Gyeonggi-do 16677 (KR); HONG, Jinwoo, Suwon-si, Gyeonggi-do 16677 (KR); HWANG, Jun, Suwon-si, Gyeonggi-do 16677 (KR); PARK, Jonghoon, Suwon-si, Gyeonggi-do 16677 (KR); LEE, Soondong, Suwon-si, Gyeonggi-do 16677 (KR)
(74) Representative: Walaski, Jan Filip
(86) International application number: PCT/KR2024/013524
(87) International publication number: WO 2025/079871

(57) **Abstract**

According to an aspect of an embodiment of the present disclosure, an air conditioner 100 is provided. The air conditioner 100 may include a detection sensor 110 configured to detect an object, an illuminance sensor 120 configured to detect illuminance, an air conditioning module 212, a communication module 216, memory 214 storing at least one instruction, and at least one processor 210. The at least one processor 210 may execute the at least one instruction to receive, from an external device 310 through the communication module 216, a sleep mode schedule input by a user, obtain, while a current time corresponds to the sleep mode schedule, an absence duration during which a person is not detected in a target space, based on a sensor detection value of the detection sensor 110, and control, based on the absence duration being greater than or equal to a sleep reference value and based on an illuminance value detected by the illuminance sensor 120 being less than an illuminance reference value, the air conditioning module to operate in a sleep mode.

## Description

### Technical Field

An embodiment of the present disclosure relates to an air conditioner for providing a sleep mode, a method of controlling an air conditioner, and a computer-readable recording medium having recorded thereon a program for causing a computer to execute the method of controlling an air conditioner.

### Background Art

Various types of air conditioners are widely used in indoor spaces. An air conditioner may include various sensors, for example, a human detection sensor, an illuminance sensor, or a temperature sensor. The air conditioner may use various sensors to regulate the environment of an air-conditioned space and control the operation of the air conditioner. Because the air conditioner adjusts the temperature and environment of an indoor space, the operation of the air conditioner has a significant influence on a user's condition. However, when individual modes provided by the air conditioner are not appropriately controlled, the modes may not be effectively utilized, or a user may experience inconvenience caused by the need to manually control each mode.

### Disclosure of Invention

### Solution to Problem

According to an aspect of an embodiment of the present disclosure, an air conditioner 100 is provided. The air conditioner 100 may include a detection sensor 110 configured to detect an object, an illuminance sensor 120 configured to detect illuminance, an air conditioning module 212, a communication module 216, memory 214 storing at least one instruction, and at least one processor 210. The at least one processor 210 may execute the at least one instruction to receive, from an external device 310 through the communication module 216, a sleep mode schedule input by a user, obtain, while a current time corresponds to the sleep mode schedule, an absence duration during which a person is not detected in a target space, based on a sensor detection value of the detection sensor 110, and control, based on the absence duration being greater than or equal to a sleep reference value and based on an illuminance value detected by the illuminance sensor 120 being less than an illuminance reference value, the air conditioning module to operate in a sleep mode.

In addition, according to an aspect of an embodiment of the present disclosure, a method of controlling an air conditioner is provided. The method of controlling an air conditioner may include receiving a sleep mode schedule input by a user, obtaining a sensor detection value of a detection sensor of the air conditioner in a home, obtaining an illuminance value detected by an illuminance sensor of the air conditioner, obtaining, while a current time corresponds to the sleep mode schedule, an absence duration during which a person is not detected in a target space, based on the sensor detection value of the detection sensor, and based on the absence duration being greater than or equal to a sleep reference value and based on the illuminance value being less than an illuminance reference value, controlling the air conditioner to operate in a sleep mode.

In addition, according to an aspect of an embodiment of the present disclosure, provided is a computer-readable recording medium having recorded thereon a program for causing a computer to execute the method of controlling an air conditioner.

### Brief Description of Drawings

An embodiment of the present disclosure may be readily understood with a combination of the following detailed descriptions and the accompanying drawings, wherein reference numbers refer to structural elements.
FIG. 1 is a diagram illustrating an operation of an air conditioner according to an embodiment of the present disclosure.
FIG. 2 is a diagram illustrating a structure of an air conditioner according to an embodiment of the present disclosure.
FIG. 3 is a diagram illustrating an air conditioner, an external device, and a server, according to an embodiment of the present disclosure.
FIG. 4 is a diagram illustrating a method of controlling an air conditioner, according to an embodiment of the present disclosure.
FIG. 5 is a diagram illustrating a process of inputting sleep mode schedule information through an external device, according to an embodiment of the present disclosure.
FIG. 6 is a diagram illustrating an operation of an air conditioning module in a sleep mode, according to an embodiment of the present disclosure.
FIG. 7 is a diagram illustrating a method of controlling an air conditioner, according to an embodiment of the present disclosure.
FIG. 8 is a diagram illustrating a structure of an air conditioner according to an embodiment of the present disclosure.
FIG. 9 is a diagram illustrating control of a lighting module in a sleep mode, according to an embodiment of the present disclosure.
FIG. 10 is a diagram illustrating a structure of an air conditioner according to an embodiment of the present disclosure.
FIG. 11 is a diagram illustrating a method of controlling an air conditioner, according to an embodiment of the present disclosure.
FIG. 12 is a view illustrating a process of stopping a sleep mode during a sleep mode operation, according to an embodiment of the present disclosure.
FIG. 13 is a diagram illustrating an air conditioner, an external device, a wearable device, and a server, according to an embodiment of the present disclosure.
FIG. 14 is a diagram illustrating an operation of controlling a sleep mode of an air conditioner, according to an embodiment of the present disclosure.
FIG. 15 is a diagram illustrating an operation of a server according to sleep mode information of an air conditioner, according to an embodiment of the present disclosure.
FIG. 16 is a diagram illustrating a process of controlling a home appliance based on sleep mode information of an air conditioner, according to an embodiment of the present disclosure.
FIG. 17 is a diagram illustrating a method of controlling an air conditioner, according to an embodiment of the present disclosure.

### Mode for the Invention

It should be appreciated that various embodiments of the present disclosure and the terms used for describing the embodiments are not intended to limit the technological features set forth herein to particular embodiments, and include various changes, equivalents, or alternatives for a corresponding embodiment.

With regard to the description of the drawings, similar reference numerals may be used to refer to similar or related elements.

A singular form of a noun corresponding to an item may include one or more of the things, unless the relevant context clearly indicates otherwise.

As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases.

As used herein, the term "and/or" includes any one or a combination of a plurality of related recited elements.

As used herein, such terms as "1st" and "2nd," or "first" and "second" may be used to simply distinguish a corresponding component from another, and does not limit the components in any other respect (e.g., importance or order).

When an element (e.g., a first element) is referred to, with or without the term "operatively" or "communicatively", as being "coupled with," "coupled to," "connected with," or "connected to" another element (e.g., a second element), it means that the element may be connected to the other element directly (e.g., in a wired manner), wirelessly, or via a third element.

As used herein, such terms as "comprises," "includes," or "has" specify the presence of stated features, numbers, stages, operations, components, parts, or a combination thereof, but do not preclude the presence or addition of one or more other features, numbers, stages, operations, components, parts, or a combination thereof.

When an element is referred to as being "connected to," "coupled to," "supported by," or "in contact with" another element, it means that the element is directly connected to, coupled to, supported by, or in contact with the other element, or that the element is indirectly connected to, coupled to, supported by, or in contact with the other element via a third element.

When an element is referred to as being "on" another element, it means that the element is in contact with the other element, or that still another element is present between the element and the other element.

An air conditioner according to an embodiment of the present disclosure is an apparatus configured to perform functions in an air-conditioned space (hereinafter referred to as an "indoor space"), such as air purification, ventilation, humidity regulation, cooling, or heating, and refers to an apparatus provided with at least one of these functions.

According to an embodiment of the present disclosure, an air conditioner may include a heat pump device to perform a cooling function or a heating function. The heat pump device may include a refrigeration cycle in which a refrigerant is circulated through a compressor, a first heat exchanger, an expansion device, and a second heat exchanger. All components of the heat pump device may be accommodated in one housing forming an exterior of the air conditioner, and examples of such an air conditioner include a window-type air conditioner or a portable air conditioner. On the other hand, some components of the heat pump device may be distributed and accommodated in a plurality of housings forming a single air conditioner, and examples of such an air conditioner include a wall-mounted air conditioner, a floor-standing air conditioner, and a system air conditioner.

An air conditioner including a plurality of housings may include at least one outdoor unit installed in an outdoor space and at least one indoor unit installed in an indoor space. For example, the air conditioner may be provided such that one outdoor unit is connected to one indoor unit via a refrigerant pipe. For example, the air conditioner may be provided such that one outdoor unit is connected to two or more indoor units via refrigerant pipes. For example, the air conditioner may be provided such that two or more outdoor units are connected to two or more indoor units via a plurality of refrigerant pipes.

An outdoor unit may be electrically connected to an indoor unit. For example, information (or a command) for controlling the air conditioner may be input through an input interface provided on the outdoor unit or the indoor unit, and the outdoor unit and the indoor unit may operate simultaneously or sequentially in response to a user input.

The air conditioner may include an outdoor heat exchanger provided in the outdoor unit, an indoor heat exchanger provided in the indoor unit, and a refrigerant pipe connecting the outdoor heat exchanger to the indoor heat exchanger.

The outdoor heat exchanger may perform heat exchange between a refrigerant and outdoor air by using a phase change (e.g., evaporation or condensation) of the refrigerant. For example, while the refrigerant is condensed in the outdoor heat exchanger, the refrigerant may release heat to the outdoor air, and while the refrigerant flowing in the outdoor heat exchanger evaporates, the refrigerant may absorb heat from the outdoor air.

The indoor unit is provided in an indoor space. For example, indoor units may be classified into ceiling-type indoor units, stand-type indoor units, wall-mounted indoor units, and the like, depending on the arrangement method thereof. For example, ceiling-type indoor units may be classified into 4-way indoor units, 1-way indoor units, and duct-type indoor units, and the like, depending on the air discharge method thereof.

Similarly, the indoor heat exchanger may perform heat exchange between a refrigerant and indoor air by using a phase change (e.g., evaporation or condensation) of the refrigerant. For example, the refrigerant may absorb heat from the indoor air while evaporating in the indoor unit, and the indoor space may be cooled by blowing the indoor air that has been cooled while passing through the cooled indoor heat exchanger. In addition, the refrigerant may release heat into the indoor air while condensing in the indoor heat exchanger, and the indoor space may be heated by blowing the indoor air that has been heated while passing through the heated indoor heat exchanger.

In other words, the air conditioner performs a cooling or heating function through a phase change process of the refrigerant circulating through the outdoor heat exchanger and the indoor heat exchanger, and for the circulation of the refrigerant, the air conditioner may include a compressor configured to compress the refrigerant. The compressor may intake refrigerant gas through a suction unit and compress the refrigerant gas. The compressor may discharge the high-temperature and high-pressure refrigerant gas through a discharge unit. The compressor may be arranged inside the outdoor unit.

The refrigerant may circulate through the refrigerant pipe sequentially through the compressor, the outdoor heat exchanger, the expansion device, and the indoor heat exchanger, or may circulate through the refrigerant pipe sequentially through the compressor, the indoor heat exchanger, the expansion device, and the outdoor heat exchanger.

For example, in a case where one outdoor unit and one indoor unit are directly connected to each other via a refrigerant pipe, the air conditioner may be provided such that the refrigerant circulates between the outdoor unit and the indoor unit through the refrigerant pipe.

For example, in a case where one outdoor unit is connected to two or more indoor units via refrigerant pipes, the refrigerant may flow to a plurality of indoor units through the refrigerant pipes branching from the outdoor unit. The refrigerant discharged from a plurality of indoor units may join together and then circulate to the outdoor unit. For example, a plurality of indoor units may be connected directly to one outdoor unit in parallel via separate refrigerant pipes, respectively.

Each of the plurality of indoor units may operate independently according to an operation mode set by a user. That is, some of the plurality of indoor units may operate in a cooling mode, while others may simultaneously operate in a heating mode. Here, the refrigerant may be selectively introduced into the respective indoor units in a high-pressure or low-pressure state along circulation paths designated through a flow path switching valve, which will be described below, and then discharged to circulate to the outdoor unit.

For example, in a case where the air conditioner has two or more outdoor units connected to two or more indoor units via a plurality of refrigerant pipes, the refrigerant discharged from a plurality of outdoor units may join together, then flow through one refrigerant pipe, and then branch out again at a certain point to be introduced into a plurality of indoor units.

All of the plurality of outdoor units may operate or at least some of them may not operate, depending on the operation load according to the operation amount of the plurality of indoor units. Here, the refrigerant may flow into and circulate in a selectively operating outdoor unit via a flow path switching valve. The air conditioner may include an expansion device to lower the pressure of the refrigerant flowing into the heat exchanger. For example, the expansion device may be arranged inside the indoor unit or inside the outdoor unit, or may be arranged inside both the indoor unit and the outdoor unit.

For example, the expansion device may lower the temperature and pressure of the refrigerant by using a throttling effect. The expansion device may include an orifice capable of reducing the cross-sectional area of a flow path. The refrigerant that has passed through the orifice may decrease in temperature and pressure.

For example, the expansion device may be implemented as an electronic expansion valve capable of adjusting an opening ratio (the ratio of the cross-sectional area of the flow path of the valve in a partially open state to the cross-sectional area of the flow path of the value in a fully open state). Depending on the opening ratio of the electronic expansion valve, the amount of refrigerant passing through the expansion device may be controlled.

The air conditioner may further include a flow path switching valve arranged on a refrigerant circulation flow path. The flow path switching valve may include, for example, a 4-way valve. The flow path switching valve may determine a circulation path of the refrigerant depending on the operation mode of the indoor unit (e.g., a cooling operation or a heating operation). The flow path switching valve may be connected to the discharge unit of the compressor.

The air conditioner may include an accumulator. The accumulator may be connected to the suction unit of the compressor. A low-temperature and low-pressure refrigerant that has evaporated in the indoor heat exchanger or the outdoor heat exchanger may flow into the accumulator.

When a mixture of refrigerant liquid and refrigerant gas flows into the accumulator, the accumulator may separate the refrigerant liquid from the refrigerant gas, and provide the compressor with the refrigerant gas from which the refrigerant liquid has been separated.

An outdoor fan may be provided adjacent to the outdoor heat exchanger. The outdoor fan may blow outdoor air toward the outdoor heat exchanger to promote heat exchange between the refrigerant and the outdoor air.

The outdoor unit of the air conditioner may include at least one sensor. For example, the sensor of the outdoor unit may be provided as an environmental sensor. The sensor of the outdoor unit may be arranged at an arbitrary location inside or outside the outdoor unit. For example, the outdoor unit sensor may include, for example, a temperature sensor configured to detect the temperature of air around the outdoor unit, a humidity sensor configured to detect the humidity of air around the outdoor unit, a refrigerant temperature sensor configured to detect the temperature of the refrigerant passing through the outdoor unit, or a refrigerant pressure sensor configured to detect the pressure of the refrigerant in the refrigerant pipe passing through the outdoor unit.

The outdoor unit of the air conditioner may include an outdoor unit communication unit. The outdoor unit communication unit may be provided to receive a control signal from a control unit of the indoor unit of the air conditioner, which will be described below. The outdoor unit may control the operation of the compressor, the outdoor heat exchanger, the expansion device, the flow path switching valve, the accumulator, or the outdoor fan based on a control signal received through the outdoor unit communication unit. The outdoor unit may transmit a sensing value detected by the outdoor unit sensor, to the control unit of the indoor unit via the outdoor unit communication unit.

The indoor unit of the air conditioner may include a housing, a blower that circulates air into or out of the housing, and an indoor heat exchanger that exchanges heat with air flowing into the housing.

The housing may include a suction port. Indoor air may flow into the housing via the suction port.

The indoor unit of the air conditioner may include a filter provided to filter out foreign substances from air flowing into the housing via the suction port.

The housing may include a discharge port. Air flowing inside the housing may be discharged to the outside of the housing via the discharge port.

The housing of the indoor unit may include an airflow guide that guides air discharged via the discharge port, in a certain direction. For example, the airflow guide may include a blade arranged on the discharge port. For example, the airflow guide may include an auxiliary fan for controlling the discharged airflow. The present disclosure is not limited thereto, and the airflow guide may be omitted.

Inside the housing of the indoor unit, an indoor heat exchanger and a blower may be arranged on a flow path connecting the suction port to the discharge port.

The blower may include an indoor fan and a fan motor. For example, the indoor fan may include an axial fan, a mixed flow fan, a cross-flow fan, and a centrifugal fan.

The indoor heat exchanger may be arranged between the blower and the discharge port, or between the suction port and the blower. The indoor heat exchanger may absorb heat from air introduced via the suction port, or transfer heat to air introduced via the suction port. The indoor heat exchanger may include a heat exchange tube through which the refrigerant flows, and a heat exchange fin in contact with the heat exchange tube to increase the heat transfer area.

The indoor unit of the air conditioner may include a drain tray arranged below the indoor heat exchanger to collect condensate generated in the indoor heat exchanger. The condensate collected in the drain tray may be discharged to the outside via a drain hose. The drain tray may be provided to support the indoor heat exchanger.

The indoor unit of the air conditioner may include an input interface. The input interface may include any type of user input unit, including buttons, switches, touch screens, and/or touch pads. The user may manually input setting data (e.g., a desired indoor temperature, operation mode settings for cooling/heating/dehumidification/air purification, an outlet selection setting, and/or an airflow rate setting) through the input interface.

The input interface may be connected to an external input device. For example, the input interface may be electrically connected to a wired remote controller. The wired remote controller may be installed at a particular location in an indoor space (e.g., a portion of a wall). The user may input setting data regarding the operation of the air conditioner by manipulating the wired remote controller. An electrical signal corresponding to the setting data obtained through the wired remote controller may be transmitted to the input interface. In addition, the input interface may include an infrared sensor. The user may remotely input setting data regarding the operation of the air conditioner by using a wireless remote controller. The setting data input through the wireless remote controller may be transmitted to the input interface as an infrared signal.

In addition, the input interface may include a microphone. A voice command of the user may be obtained through the microphone. The microphone may convert the voice command of the user into an electrical signal and transmit the electrical signal to an indoor unit control unit. The indoor unit control unit may control the components of the air conditioner to execute a function corresponding to the voice command of the user. The setting data (e.g., a desired indoor temperature, operation mode settings for cooling/heating/dehumidification/air purification, an outlet selection setting, and/or an airflow rate setting) obtained through the input interface may be delivered to the indoor unit control unit, which will be described below. In an example, setting data obtained through the input interface may be transmitted to the outside, that is, the outdoor unit or a server, via an indoor unit communication unit, which will be described below.

The indoor unit of the air conditioner may include a power module. The power module may be connected to an external power source to supply power to the components of the indoor unit.

The indoor unit of the air conditioner may include an indoor unit sensor. The indoor unit sensor may be an environmental sensor arranged inside or outside the housing. For example, the indoor unit sensor may include one or more temperature sensors and/or humidity sensors arranged in a predetermined space inside or outside the housing of the indoor unit. For example, the indoor unit sensor may include a refrigerant temperature sensor configured to detect the temperature of a refrigerant in the refrigerant pipe passing through the indoor unit. For example, the indoor unit sensor may include refrigerant temperature sensors configured to detect the temperature of an inlet, a middle point, and/or an outlet of the refrigerant pipe passing through the indoor heat exchanger.

For example, each environmental information detected by the indoor unit sensor may be delivered to the indoor unit control unit, which will be described below, or may be transmitted to the outside through the indoor unit communication unit, which will be described below.

The indoor unit of the air conditioner may include an indoor unit communication unit. The indoor unit communication unit may include at least one of a short-range wireless communication module or a long-range communication module. The indoor unit communication unit may include at least one antenna for wireless communication with other devices. The outdoor unit may include an outdoor unit communication unit. The outdoor unit communication unit may include at least one of a short-range wireless communication module or a long-range communication module.

The short-range wireless communication module may include, but is not limited to, a Bluetooth communication module, a Bluetooth Low Energy (BLE) communication module, a near-field communication module, a wireless local area network (WLAN) (Wi-Fi) communication module, a Zigbee communication module, an IrDA communication module, a Wi-Fi Direct (WFD) communication module, an ultra-wideband (UWB) communication module, an Ant+ communication module, a microwave (uWave) communication module, and the like.

The long-range communication module may include a communication module configured to perform various types of long-range communication, and may include a mobile communication unit. The mobile communication unit transmits and receives wireless signals to and from at least one of a base station, an external terminal, or a server, on a mobile communication network.

The indoor unit communication unit may communicate with external devices such as servers, mobile devices, or other home appliances, via a nearby access point (AP). The AP may connect a local area network (LAN) to which the air conditioner or a user device is connected, to a wide area network (WAN) to which a server is connected. The air conditioner or the user device may be connected to the server via the WAN. The indoor unit of the air conditioner may include an indoor unit control unit configured to control the components of the indoor unit, such as the blower. The outdoor unit of the air conditioner may include an outdoor unit control unit configured to control the components of the outdoor unit, such as the compressor. The indoor unit control unit may communicate with the outdoor unit control unit via the indoor unit communication unit and the outdoor unit communication unit. The outdoor unit communication unit may transmit, to the indoor unit communication unit, a control signal generated by the outdoor unit control unit, or may deliver, to the outdoor unit control unit, a control signal transmitted from the indoor unit communication unit. That is, the outdoor unit and the indoor unit may perform bidirectional communication. The outdoor unit and the indoor unit may transmit and receive various signals generated during the operation of the air conditioner.

The outdoor unit control unit may be electrically connected to the components of the outdoor unit, and may control the operation of each component. For example, the outdoor unit control unit may adjust the frequency of the compressor, and may control the flow path switching valve to change the circulation direction of the refrigerant. The outdoor unit control unit may adjust the rotational speed of an outdoor fan. In addition, the outdoor unit control unit may generate a control signal for adjusting the opening degree of the expansion valve. Under control of the outdoor unit control unit, the refrigerant may circulate along a refrigerant circulation circuit including the compressor, the flow path switching valve, the outdoor heat exchanger, the expansion valve, and the indoor heat exchanger.

Various temperature sensors included in the outdoor unit and the indoor unit may transmit electrical signals corresponding to respective detected temperatures to the outdoor unit control unit and/or the indoor unit control unit. For example, the humidity sensors included in the outdoor unit and the indoor unit may transmit electrical signals corresponding to respective detected humidities to the outdoor unit control unit and/or the indoor unit control unit.

The indoor unit control unit may obtain a user input from a user device, such as a mobile device, via the indoor unit communication unit, and may obtain a user input directly through the input interface or through a remote controller. In response to the received user input, the indoor unit control unit may control the components of the indoor unit, such as the blower. The indoor unit control unit may transmit information about the received user input to the outdoor unit control unit of the outdoor unit.

The outdoor unit control unit may control the components of the outdoor unit, such as the compressor, based on the information about the user input received from the indoor unit. For example, in response to receiving, from the indoor unit, a control signal corresponding to a user input for selecting an operation mode such as a cooling operation, a heating operation, a blowing operation, a defrosting operation, or a dehumidification operation, the outdoor unit control unit may control the components of the outdoor unit to perform an operation of the air conditioner corresponding to the selected operation mode.

The outdoor unit control unit and the indoor unit control unit may each include a processor and memory. The indoor unit control unit may include at least one first processor and at least one first memory, and the outdoor unit control unit may include at least one second processor and at least one second memory.

The memory may store various pieces of information necessary for the operation of the air conditioner. The memory may store instructions, applications, data, and/or programs necessary for the operation of the air conditioner. For example, the memory may store various programs for a cooling operation, a heating operation, a dehumidification operation, and/or a defrosting operation of the air conditioner. The memory may include volatile memory for temporarily storing data, such as static random-access memory (S-RAM) or dynamic RAM (D-RAM). In addition, the memory may include non-volatile memory for long-term storage of data, such as read-only memory (ROM), erasable programmable ROM (EPROM), or electrically erasable programmable ROM (EEPROM).

Based on instructions, applications, data, and/or programs stored in the memory, the processor may generate a control signal for controlling the operation of the air conditioner. The processor, as a hardware component, may include logic circuitry and arithmetic circuitry. The processor may process data according to programs and/or instructions provided from the memory, and generate a control signal according to a result of the processing. The memory and the processor may be implemented as a single piece of control circuitry or as a plurality of pieces of circuitry.

The indoor unit of the air conditioner may include an output interface. The output interface may be electrically connected to the indoor unit control unit, and may output information related to the operation of the air conditioner under control of the indoor unit control unit. For example, the output interface may output information such as an operation mode, an airflow direction, an airflow rate, or a temperature selected by a user input. In addition, the output interface may output sensing information obtained from the indoor unit sensor or the outdoor unit sensor, and warning/error messages.

The output interface may include a display and a speaker. The speaker is an audio device capable of outputting various sounds. The display may display information input by the user or information to be provided to the user, by using various graphical elements. For example, operation information of the air conditioner may be displayed as at least one of an image or text. In addition, the display may include an indicator configured to provide particular information. The display may include a liquid-crystal display (LCD) panel, a light-emitting diode (LED) panel, an organic light-emitting diode (OLED) panel, a micro-LED panel, and/or a plurality of LEDs.

Hereinafter, an air conditioner according to various embodiments will be described in detail with reference to the drawings.

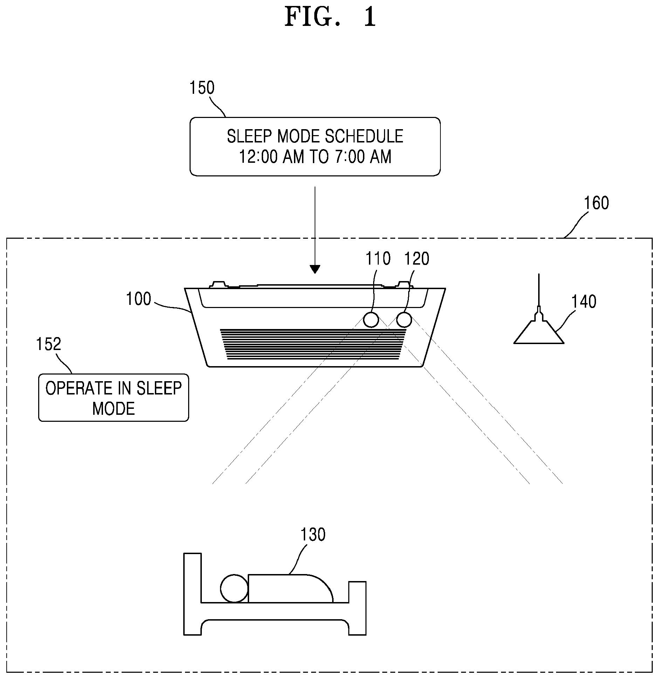

FIG. 1 is a diagram illustrating an operation of an air conditioner according to an embodiment of the present disclosure.

According to an embodiment of the present disclosure, an air conditioner 100 performs an air conditioning operation for a target space 160. The air conditioning operation may include, for example, cooling, heating, air purification, dehumidification, or blowing. The air conditioner 100 may be implemented in the form of a cooling apparatus, a heating apparatus, a cooling and heating apparatus, an air purifier, or a dehumidifier. The present disclosure primarily describes a case where the air conditioner 100 corresponds to a cooling apparatus. However, this is for convenience of description, and embodiments of the present disclosure are not limited thereto.

The air conditioner 100 may include a detection sensor 110. The detection sensor 110 detects an object in the target space 160. The air conditioner 100 may detect a motion value of a user 130 in the target space 160 by using a sensor detection value of the detection sensor 110. By using the motion value of the user 130 in the target space 160, the air conditioner 100 may detect an absence duration during which motion of the user 130 is not detected.

In addition, the air conditioner 100 may include an illuminance sensor 120. The illuminance sensor 120 detects the illuminance of the target space 160. The air conditioner 100 may identify or obtain an illuminance value by using a sensor detection value of the illuminance sensor 120. The illuminance value may vary depending on an on/off state of a lighting device 140 in the target space 160, a set illuminance, daylighting, or the like.

The target space 160 refers to an indoor space in which the air conditioner 100 may be installed. The target space 160 may correspond to various types of indoor spaces, such as a house, an office, a shop, a guest room, a commercial space, or a work space.

In operation 150, the air conditioner 100 may receive sleep mode schedule information of the user 130. The sleep mode schedule information refers to information indicating a sleep schedule of the user 130. The sleep mode schedule information may include at least one of a bedtime, a sleep duration, or a wake-up time. The sleep mode schedule information may be input through an external device (e.g., a smart phone) or may be input through an input interface of the air conditioner 100.

When the current time corresponds to the sleep mode schedule, in operation 152, the air conditioner 100 may automatically enter and operate in a sleep mode based on the absence duration and the illuminance value. When the user 130 is sleeping, the motion value is significantly low because there is almost no motion. Accordingly, when the user 130 is sleeping, the air conditioner 100 may determine that the user 130 is absent, based on a sensor detection value of the detection sensor 110. In addition, when the user 130 is sleeping, the illuminance of the target space 160 may be set to a low level by turning off the lighting device 140. Thus, when the user 130 is sleeping, the illuminance of the target space 160 may be set to a significantly low level. However, a condition in which the user is absent and the illuminance value is low may also occur even when the user 130 is actually absent. According to an embodiment of the present disclosure, it is possible for the air conditioner 100 to accurately recognize a situation in which the user 130 is sleeping with a high probability by obtaining sleep mode schedule information in advance and determining whether the user 130 is sleeping based on the absence duration and the illuminance value during a time period corresponding to the sleep mode schedule.

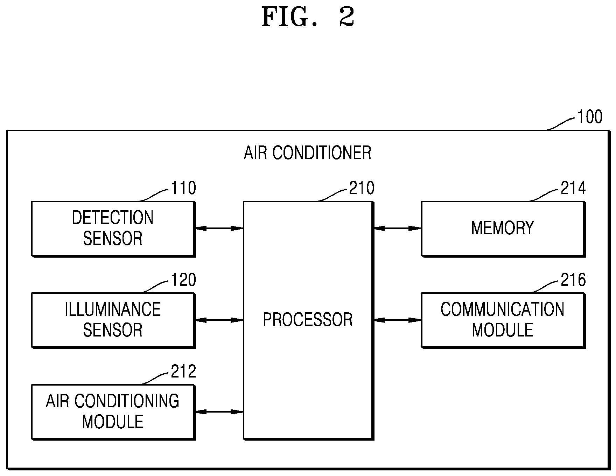

FIG. 2 is a diagram illustrating a structure of an air conditioner according to an embodiment of the present disclosure.

According to an embodiment of the present disclosure, the air conditioner 100 includes the detection sensor 110, the illuminance sensor 120, a processor 210, an air conditioning module 212, memory 214, and a communication module 216. The block diagram of the air conditioner 100 of FIG. 2 may correspond to a block diagram of an indoor unit.

The air conditioner 100 may be implemented in various installation forms. For example, the air conditioner 100 may be implemented in the form of a floor-standing air conditioner, a wall-mounted air conditioner, a ceiling-embedded system air conditioner, or a home multi-air conditioner.

The detection sensor 110 may detect an object in the target space 160. The detection sensor 110 may include, for example, a time-of-flight (ToF) sensor, an ultrasonic sensor, an infrared sensor, an optical sensor, a camera, a radio detection and ranging (RADAR) sensor, a light detection and ranging (LiDAR) sensor, or the like. The detection sensor 110 is arranged to output a signal to the target space 160 and detect a reflected signal. The detection sensor 110 may be arranged on a front surface of the air conditioner 100 toward the target space 160. The detection sensor 110 generates a sensor detection value and transfers the sensor detection value to the processor 210.

The illuminance sensor 120 is a sensor configured to measure the illuminance of the target space 160. The illuminance sensor 120 may include, for example, a photoresistor, a photodiode, a phototransistor, or the like. The illuminance sensor 120 may be arranged on the front surface of the air conditioner 100 toward the target space 160 to detect the illuminance of the target space 160. The illuminance sensor 120 may generate an illuminance value and transfer the illuminance value to the processor 210.

The processor 210 controls the overall operation of the air conditioner 100. The processor 210 may be implemented as one or more processors. The processor 210 may perform a predetermined operation by executing instructions or commands stored in the memory 214. In addition, the processor 210 controls the operation of components provided in the air conditioner 100. The processor 210 may include a central processing unit (CPU), a microprocessor, and the like.

The processor 210 determines whether a moving object is present by using a sensor detection value of the detection sensor 110, and when a moving object is present, determines that a person is present in the target space 160. According to an embodiment of the present disclosure, the processor 210 determines whether the detected object is a person, by using the sensor detection value. For example, in a case where the detection sensor 110 corresponds to an infrared sensor, when an infrared value corresponding to a person is detected, the processor 210 determines that a person is present in the target space 160. According to an embodiment of the present disclosure, the processor 210 determines whether the detected object has a shape of a person based on the sensor detection value, and when the detected object corresponds to a shape of a person, determines that a person is present in the target space 160.

By using the sensor detection value of the detection sensor 110, when no moving object is present in the target space 160, the processor 210 determines that a person is not present in the target space 160. The processor 210 counts, as an absence duration, a duration during which it is determined that a person is not present. The absence duration refers to a duration during which a person is not present. When a person is detected again during counting of the absence duration, the absence duration is reset to 0. When a motion value detected by the sensor detection value of the detection sensor 110 is less than a reference value, the processor 210 may determine that a person is not present and count the absence duration.

According to an embodiment of the present disclosure, the detection sensor 110 may correspond to a radar sensor, and the processor 210 may determine whether the detected object has a shape of a person by using a sensor detection value of the radar sensor. The radar sensor outputs a radar signal to the target space 160 and detects, as a sensor detection value, a signal reflected from an object in the target space 160. The processor 210 detects an object in the target space 160 by using a sensor detection value of the radar sensor. The processor 210 detects an object in the target space 160 at a predetermined frame rate and detects a motion of the object. When a motion value of the object in the target space 160 is greater than or equal to a reference value, the processor 210 determines that a person is present in the target space 160. For example, the processor 210 detects an object in the target space 160 at a rate of 30 frames/sec, and when a motion value per second of the object is greater than or equal to a reference value, determines that a person is present in the target space 160.

In addition, according to an embodiment of the present disclosure, the processor 210 determines whether the recognized object is a person, based on a result of object recognition based on a sensor detection value of the radar sensor. The processor 210 may determine whether the recognized object is a person, based on a shape of the recognized object. When the recognized object corresponds to a person and the motion value is greater than or equal to the reference value, the processor 210 determines that a person is present in the target space 160. When it is determined that the recognized object does not correspond to a person, the processor 210 determines that a person is not present in the target space 160. In addition, according to an embodiment of the present disclosure, even when the recognized object corresponds to a pet, the processor 210 may determine that a person is present in the target space 160. Accordingly, when the detected object corresponds to a person or a pet and the motion value is greater than or equal to the reference value, the processor 210 may determine that a person is present in the target space 160.

The processor 210 obtains an illuminance value from the illuminance sensor 120. According to an embodiment of the present disclosure, the processor 210 may obtain an illuminance value by performing predetermined processing on a sensor detection value from the illuminance sensor 120. The processor 210 determines whether the illuminance value is less than an illuminance reference value. The illuminance reference value may be, for example, 25 lux.

The air conditioning module 212 performs an air conditioning operation. The air conditioning module 212 adjusts activation of cooling, cooling intensity, activation of heating, heating intensity, an airflow rate, and the like, based on a control signal or a driving signal input from the processor 210. The air conditioning module 212 may include a heat exchanger, a motor, an inverter, a fan, a filter, and the like. The air conditioning module 212 may include the heat exchanger and may perform heat exchange between a refrigerant and indoor air by using a phase change (e.g., expansion or compression) of the refrigerant in the heat exchanger. For example, while the refrigerant expands in the heat exchanger, the refrigerant may absorb heat from the indoor air, and the indoor space may be cooled. While the refrigerant is compressed in the heat exchanger, the refrigerant may release heat to the indoor air, and the indoor space may be heated.

The memory 214 stores various pieces of information, data, instructions, programs, and the like necessary for the operation of the air conditioner 100. The memory 214 may include at least one of volatile memory or non-volatile memory, or a combination thereof. The memory 214 may include at least one of a flash memory-type storage medium, a hard disk-type storage medium, a multimedia card micro-type storage medium, a card-type memory (e.g., Secure Digital (SD) or eXtreme Digital (XD) memory), RAM, static RAM (SRAM), ROM, electrically erasable programmable ROM (EEPROM), programmable ROM (PROM), magnetic memory, a magnetic disk, and an optical disc. In addition, the memory 214 may correspond to a web storage or a cloud server that performs a storage function on the Internet.

The communication module 216 may communicate with at least one external device in a wired or wireless manner. According to an embodiment of the present disclosure, the communication module 216 perform wireless communication with a remote controller. The communication module 216 may receive, from the remote controller, a power on/off signal, a temperature setting signal, an operation mode selection signal, a blowing intensity selection signal, a sleep scheduling signal, a sleep mode control signal, a scheduled operation setting signal, an airflow direction setting signal, or the like. The communication module 216 may transmit status information of the air conditioner 100 to the remote controller to synchronize the status information between the air conditioner 100 and the remote controller.

In addition, according to an embodiment of the present disclosure, the communication module 216 may communicate with an outdoor unit. For example, the communication module 216 may communicate with the outdoor unit by using RS-485 serial communication.

In addition, according to an embodiment of the present disclosure, the communication module 216 may communicate with a server via a network. The communication module 216 may connect to the network through an AP device, and communicate with the server. The communication module 216 may receive sleep mode schedule information from the server. In addition, the communication module 216 may receive, from the server, a power on/off signal, a temperature setting signal, an operation mode selection signal, a blowing intensity selection signal, a sleep scheduling signal, a sleep mode setting signal, a scheduled operation setting signal, an airflow direction setting signal, or the like. The communication module 216 may transmit status information of the air conditioner 100 to the server to synchronize the state information between the server and the air conditioner 100. In addition, the communication module 216 may receive, from the server, an operation mode or setting information of the air conditioner 100, which is set by using a user terminal or the like.

The communication module 216 may include a wireless communication module (e.g., a cellular communication module, a short-range wireless communication module, or a global navigation satellite system (GNSS) communication module) or a wired communication module (e.g., a LAN communication module or a power line communication module). In addition, the communication module 216 may perform short-range communication, and may use, for example, Bluetooth, BLE, near-field communication, WLAN (Wi-Fi), Zigbee, IrDA communication, WFD, UWB, Ant+ communication, or the like. In addition, for example, the communication module 216 may perform long-range communication, and may communicate with an external device through, for example, a legacy cellular network, a 5G network, a next-generation communication network, the Internet, a computer network (e.g., a LAN or a WAN), or the like.

In addition, for example, the communication module 216 may use mobile communication, and may transmit and receive wireless signals to and from at least one of a base station, an external terminal, and a server, on a mobile communication network.

According to an embodiment of the present disclosure, the communication module 216 is connected to an AP inside a home through Wi-Fi communication. The communication module 216 may communicate with an external device through the AP.

The processor 210 may receive sleep mode schedule information through the communication module 216.

According to an embodiment of the present disclosure, the sleep mode schedule information may be input through an external device (e.g., a smart phone). The external device may execute a program or an application for controlling the air conditioner 100. When a user inputs sleep mode schedule information through an input interface, the external device transmits the input sleep mode schedule information to a server. When the server receives the sleep mode schedule information, the server may transmit the sleep mode schedule information to the air conditioner 100.

According to an embodiment of the present disclosure, the sleep mode schedule information may be input through an input interface of the air conditioner 100. The air conditioner 100 may receive the sleep mode schedule information through a remote controller or an input interface of an indoor unit body.

When the current time corresponds to a sleep mode schedule, the processor 210 may determine whether to enter a sleep mode, based on an absence duration and an illuminance value by using the received sleep mode schedule information.

The processor 210 counts an absence duration during which it is determined that a person is not present in the target space 160. The processor 210 counts, as the absence duration, a time period during which it is continuously determined that a person is not present. When a person is detected in the target space 160, the absence duration is reset to 0. Thereafter, when it is determined again that a person is not present in the target space 160, the absence duration is counted again from 0.

When the current time corresponds to a sleep mode schedule, the absence duration is greater than or equal to a sleep reference value, and an illuminance value is less than an illuminance reference value, the processor 210 operates in a sleep mode. The sleep reference value may be, for example, in a range of 30 minutes to 1 hour.

In the sleep mode, the processor 210 may control the air conditioning module 212 to operate with preset parameters. In the sleep mode, a target temperature of the air conditioner is adjusted according to a sleep cycle of the user. The sleep mode may include a sleep initiation mode, a sound-sleep mode, and a wake-up mode. In each mode of the sleep mode, a target temperature is set over time by applying a predetermined temperature offset to a preset user-set temperature. In addition, the sleep mode may be performed for a preset sleep duration.

To adjust a target temperature, the processor 210 may adjust the target temperature by changing a temperature setting value of an indoor unit and adjusting a motor rotation speed of an outdoor unit compressor or an indoor unit compressor. For example, when a set temperature is set by the user, the processor 210 may adjust the revolutions per minute (RPM) of the motor according to the set temperature. When an indoor temperature detected by a temperature sensor is higher than the target temperature, the processor 210 may control the RPM of the motor of the compressor to increase, and when the indoor temperature detected by the temperature sensor is lower than the target temperature, the processor 210 may decrease the RPM of the motor of the compressor or stop the motor of the compressor. The processor 210 may generate a control signal for adjusting the RPM of the motor of the compressor, and output the control signal to the air conditioning module 212. The air conditioning module 212 may adjust the degree of cooling of air by adjusting the RPM of the motor of the compressor according to the control signal from the processor 210. The indoor temperature may follow the target temperature by adjusting the RPM of the motor of the compressor. By increasing the RPM of the motor of the compressor, the air conditioning module 212 may discharge an airflow having a lower temperature than before into the indoor space, thereby decreasing the indoor temperature. In addition, by reducing the RPM of the motor of the compressor or stopping the motor of the compressor, the air conditioning module 212 may discharge an airflow with a higher temperature than before into the indoor space, thereby increasing the indoor temperature.

The processor 210 may switch to a windless mode by controlling an airflow gate or a blade of the indoor unit to adjust airflow intensity. The indoor unit operates in the windless mode by discharging air with the wind door closed. The processor 210 generates a control signal for closing the airflow gate and outputs the control signal to the air conditioning module 212. In response to the control signal input from the processor 210, the air conditioning module 212 closes the airflow gate to operate in the windless mode. In addition, the processor 210 may control a fan speed of the indoor unit to adjust airflow intensity. The processor 210 generates a control signal for adjusting the fan speed and outputs the control signal to the air conditioning module 212. In response to the control signal input from the processor 210, the air conditioning module 212 adjusts the fan speed.

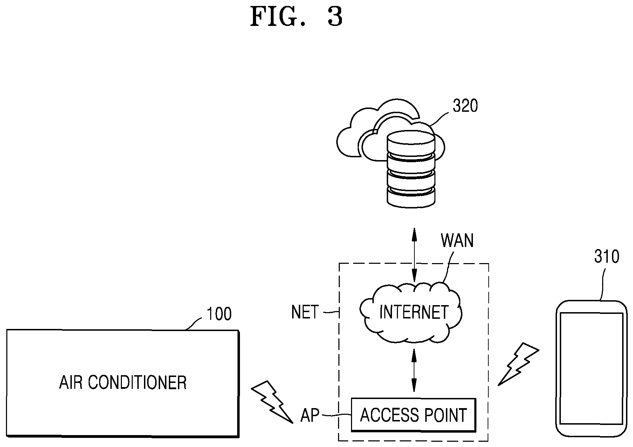

FIG. 3 is a diagram illustrating an air conditioner, an external device, and a server, according to an embodiment of the present disclosure.

According to an embodiment of the present disclosure, the air conditioner 100 communicates with an external device 310 and a server 320 via the communication module 216. The air conditioner 100 may be connected to other home appliances, the external device 310, or the server 320, via a network NET.

The server 320 may manage user account information and information about the air conditioner 100 associated with the user account. For example, a user may connect to the server 320 via the external device 310 to create a user account. The user account may be identified by an identifier (ID) and a password both set by the user. The server 320 may register the air conditioner 100 to the user account according to a predetermined procedure. For example, the server 320 may register the air conditioner 100 by associating identification information (e.g., a serial number or a medium access control (MAC) address) of the air conditioner 100, with the user account.

The external device 310 may include a communication module capable of communicating with the air conditioner 100 and the server 320, a user interface configured to receive a user input or output information to the user, at least one processor configured to control the operation of the external device 310, and at least one memory storing a program for controlling the operation of the external device 310.

The external device 310 may be carried by the user or may be arranged in the user's residence, an office, or the like. The external device 310 may include, for example, a personal computer, a terminal, a portable telephone, a smart phone, a handheld device, a wearable device, or the like, but is not limited thereto.

A program (e.g., an application) for controlling the air conditioner 100 may be stored in the memory of the external device 310. The external device 310 may be sold with an application for controlling the air conditioner 100 installed thereon, or may be sold without the application. In a case where the external device 310 is sold without an application for controlling the air conditioner 100, the user may download the application from an external server that provides applications and install it on the external device 310.

The user may control the air conditioner 100 by using the application installed on the external device 310. For example, when the user executes the application installed on the external device 310, identification information of the air conditioner 100 associated with the same user account as the external device 310 may be shown in an application execution window. The user may perform desired control on the air conditioner 100 through the application execution window. When the user inputs a control command for the air conditioner 100 through the application execution window, the external device 310 may transmit the control command directly to the air conditioner 100 through a LAN, or may transmit the control command to the air conditioner 100 via the server 320.

The application of the external device 310 may receive various user inputs for controlling the air conditioner 100. The application provides a graphical user interface (GUI) to receive various user inputs, and receives a user input through the GUI. The external device 310 communicates with the server 320 to update status information of the air conditioner 100, and provide the status information via the application. In addition, the external device 310 communicates with the server 320 to transmit, to the air conditioner 100, a user input received through the application.

The application may receive a power-off signal or a termination reservation signal for the air conditioner 100. In addition, the application may receive a reservation setting signal and receive a user input for setting a reservation end time. In addition, the application may receive a sleep mode setting signal and receive a user input for setting a reservation end time. In addition, the application may receive a user input for setting a noise prevention mode. In addition, the application may receive a user input for setting an automatic drying function. In addition, the application may receive a user input for setting a windless mode.

In addition, the application may receive a user input for selecting a user-specified mode. In addition, according to an embodiment of the present disclosure, the application may receive a user input for setting an absence schedule.

The network NET may include both a wired network and a wireless network. The wired network may include a cable network or a telephone network, and the wireless network may include any network for transmitting and receiving signals through radio waves. The wired network and the wireless network may be connected to each other.

The network NET may include a WAN such as the Internet, a LAN centered on an AP, and a wireless personal area network (WPAN) that does not pass through an AP. The short-range wireless network may include Bluetooth^{™} (Institute of Electrical and Electronics Engineers (IEEE) 802.15.1), Zigbee (IEEE 802.15.4), WFD, near-field communication (NFC), Z-Wave, and the like, but is not limited thereto.

The AP may connect a LAN to which the air conditioner 100 and the external device 310 are connected, to a WAN to which the server 320 is connected. The air conditioner 100 or the external device 310 may be connected to the server 320 via the WAN.

The AP may include a device that allows devices to connect to each other by using related standards using Wi-Fi in a computer network.

According to embodiments of the present disclosure, the AP may include an AP implemented in a hardware manner, and an AP implemented in a software manner.

For example, the AP may relay data between a wireless device and a wired device on a network. However, the present disclosure is not limited thereto, and the AP may relay data between wired devices or between wireless devices. In addition, the AP may also be referred to as a relay device.

The AP may communicate with the air conditioner 100 and the external device 310 by using wireless communication such as Wi-Fi^{™} (IEEE 802.11), and may connect to a WAN by using wired communication.

The air conditioner 100 may transmit information about an operation or a state to the server 320 via the network NET. For example, the air conditioner 100 may transmit information about an operation or a state to the server 320 through Wi-Fi^{™} (IEEE 802.11) communication.

In a case where the air conditioner 100 is not equipped with a Wi-Fi communication module, the air conditioner 100 may transmit information about an operation or a state to the server 320 via another home appliance having a Wi-Fi communication module. For example, when the air conditioner 100 transmits information about an operation or a state to another home appliance via a short-range wireless network (e.g., BLE communication), the other home appliance may transmit the information about the operation or state of the air conditioner 100 to the server 320. In addition, for example, in a case where the air conditioner 100 is not equipped with a Wi-Fi communication module, the air conditioner 100 may be connected to a communication relay device in a wired manner, and perform Wi-Fi communication and RS-485 communication through the communication relay device.

The air conditioner 100 may provide information about an operation or a state of the air conditioner 100 to the server 320 according to the user's prior approval. Information transmission to the server 320 may occur when a request is received from the server 320, may occur when a particular event occurs in the air conditioner 100, or may occur periodically or in real time.

When the server 320 receives information about an operation or a state from the air conditioner 100, the server 320 may update previously stored information related to the air conditioner 100. The server 320 may transmit information about an operation or a state of the air conditioner 100 to the external device 310 via the network NET.

When a request is received from the external device 310, the server 320 may transmit information about an operation or a state of the air conditioner 100 to the external device 310. For example, when the user executes, on the external device 310, an application connected to the server 320, the external device 310 may request and receive information about an operation or a state of the air conditioner 100 from the server 320 through the application. When information about an operation or a state is received from the air conditioner 100, the server 320 may transmit the information about the operation or state of the air conditioner 100 to the external device 310 in real time. The server 320 may periodically transmit information about an operation or a state of the air conditioner 100 to the external device 310. The external device 310 may deliver information about an operation or a state of the air conditioner 100 to the user by displaying the information about the operation or state of the air conditioner 100 on an application execution window.

The air conditioner 100 may obtain various pieces of information from the server 320 and provide the obtained information to the user. In addition, the air conditioner 100 may receive, from the server 320, a file for updating pre-installed software or data related to the pre-installed software, and update the pre-installed software or the data related to the pre-installed software based on the received file.

The air conditioner 100 may operate according to a control command received from the server 320. For example, when the air conditioner 100 obtains prior approval from the user to operate according to a control command of the server 320 even without a user input, the air conditioner 100 may operate according to a control command received from the server 320. The control command received from the server 320 may include a control command input by the user through the external device 310 or a control command generated by the server 320 based on preset conditions, but is not limited thereto.

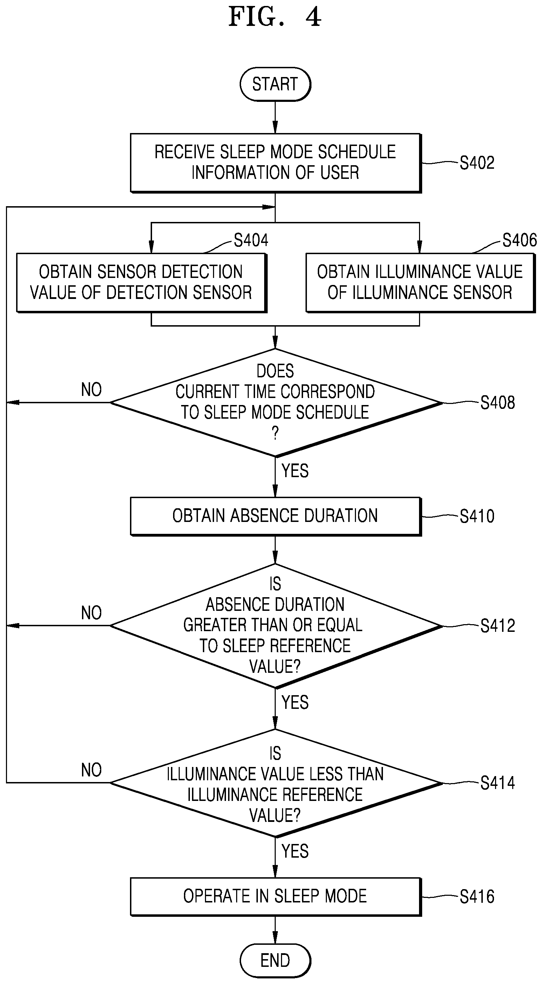

FIG. 4 is a diagram illustrating a method of controlling an air conditioner, according to an embodiment of the present disclosure.

According to an embodiment of the present disclosure, the method of controlling an air conditioner may be performed by the air conditioner 100. In addition, according to an embodiment of the present disclosure, the method of controlling an air conditioner may be performed by the server 320 communicating with the air conditioner 100. In the present disclosure, descriptions will focus on an embodiment in which the air conditioner 100 performs the method of controlling an air conditioner. However, embodiments of the present disclosure are not limited thereto, and the embodiments of the present disclosure are also applicable to a case where the server 320 performs the method of controlling an air conditioner.

Referring to FIG. 4, in operation S402, the air conditioner 100 receives sleep mode schedule information of a user.

According to an embodiment of the present disclosure, the sleep mode schedule information may be received through the external device 310. For example, the sleep mode schedule information may be received from the user through an application executed by the external device 310. The external device 310 may transmit the sleep mode schedule information to the server 320, and the server 320 may transmit the sleep mode schedule information to the air conditioner 100. The air conditioner 100 may receive and store the sleep mode schedule information from the server 320.

According to an embodiment of the present disclosure, the sleep mode schedule information may be input through an input interface of the air conditioner 100.

Operation S402 may be performed at an arbitrary time point during an operation of the air conditioner 100. Operation S402 may be performed at an arbitrary time point at which the sleep mode schedule information is received from the user.

The sleep mode schedule information may be added, modified, or deleted after being initially registered. When the sleep mode schedule information is added, modified, or deleted, the air conditioner 100 may receive the added, modified, or deleted sleep mode schedule information, and update and store the sleep mode schedule information.

Next, in operation S404, the air conditioner 100 obtains a sensor detection value of the detection sensor 110. In addition, in operation S406, the air conditioner 100 obtains an illuminance value of the illuminance sensor 120. Operations S404 and S406 may be performed in parallel, or may be sequentially performed according to a predetermined order.

Next, in operation S408, the air conditioner 100 determines whether the current time corresponds to a sleep mode schedule. A case where the sleep mode schedule is from 11:30 PM to 6:30 AM will be described as an example. When the current time is 11:50 PM, the air conditioner 100 may determine that the current time corresponds to the sleep mode schedule. When the current time is 10:00 PM, the air conditioner 100 may determine that the current time does not correspond to the sleep mode schedule.

According to an embodiment of the present disclosure, when the current time is within a reference time period from the start time of the sleep mode schedule, the air conditioner 100 may determine that the current time corresponds to the sleep mode schedule. For example, it is assumed that the sleep mode schedule is from 11:30 PM to 6:30 AM and the reference time period is 30 minutes. When the current time is 11:10 PM, because the current time is within the reference time period from 11:30 PM, which is the start time of the sleep mode schedule, the air conditioner 100 may determine that the current time corresponds to the sleep mode schedule.

When it is determined that the current time does not correspond to the sleep mode schedule, the air conditioner 100 continues to obtain a sensor detection value of the detection sensor 110 in operation S404 and obtain an illuminance value of the illuminance sensor 120 in operation S406.

When it is determined that the current time corresponds to the sleep mode schedule, in operation S410, the air conditioner 100 obtains an absence duration. According to an embodiment of the present disclosure, the air conditioner 100 may detect a motion value in a target space based on a sensor detection value of the detection sensor 110. The air conditioner 100 may count, as the absence duration, a duration during which the motion value continuously remains less than a reference value. When the motion value is detected to be greater than or equal to the reference value during counting of the absence duration, the counting of the absence duration is stopped and the absence duration is reset to 0.

Next, in operation S412, the air conditioner 100 determines whether the absence duration is greater than or equal to a sleep reference value. When the absence duration is greater than or equal to the sleep reference value, in operation S414, the air conditioner 100 determines whether the illuminance value is less than an illuminance reference value. The order of operation S412 and operation S414 is not limited to the order illustrated in FIG. 4. The air conditioner 100 may determine whether the absence duration is greater than or equal to the sleep reference value, and whether the illuminance value is less than the illuminance reference value, in various orders.

When the absence duration is greater than or equal to the sleep reference value and the illuminance value is less than the illuminance reference value, in operation S416, the air conditioner 100 operates in a sleep mode. In the sleep mode, the air conditioner 100 may control the operation of the air conditioner 100 according to preset parameters and timing. According to an embodiment of the present disclosure, in the sleep mode, the air conditioner 100 may control an air conditioning operation of the air conditioning module 212 according to preset parameters and timing. In addition, according to an embodiment of the present disclosure, in the sleep mode, the air conditioner 100 may control a lighting module of the air conditioner 100. In addition, according to an embodiment of the present disclosure, the air conditioner 100 may transmit information that the air conditioner 100 operates in the sleep mode to the server 310, and the server 310 may control other home appliances to operate in a sleep mode.

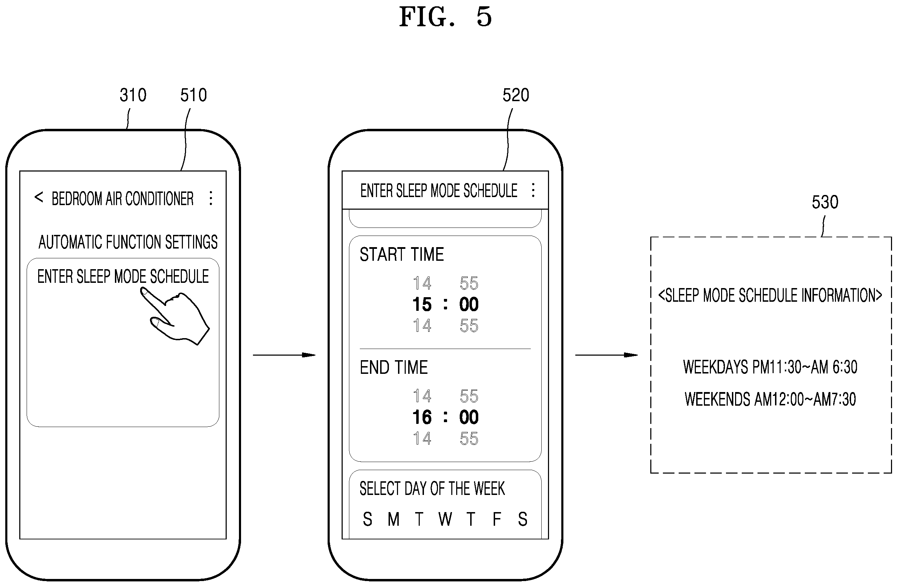

FIG. 5 is a diagram illustrating a process of inputting sleep mode schedule information through an external device, according to an embodiment of the present disclosure.

According to an embodiment of the present disclosure, a user may input a sleep mode schedule of the user through an application of the external device 310. The air conditioner 100 may receive, through the server 320, the sleep mode schedule of the user, which is input through the application of the external device 310.

According to an embodiment of the present disclosure, the application of the external device 310 provides a first GUI view 510 for inputting a sleep mode schedule. The user may select a menu item for inputting a sleep mode schedule, through the first GUI view 510.

According to an embodiment of the present disclosure, the sleep mode schedule may be a schedule in which the user is expected to sleep. That is, the sleep mode schedule is not limited to the air conditioner 100 and may correspond to a sleep mode schedule applied to all home appliances within a home registered in the server 320. The air conditioner 100 may obtain sleep mode schedule information from the sleep mode schedule of the user.

In addition, according to an embodiment of the present disclosure, the sleep mode schedule may be a sleep mode schedule for the air conditioner 100. The user may individually input a sleep mode schedule for each home appliance.

When the menu item for inputting a sleep mode schedule is selected, the external device 310 provides a menu item for selecting a sleep mode schedule in a second GUI view 520. The external device 310 may receive a start time, an end time, a day of the week, a date, or the like of the sleep mode schedule through the second GUI view 520. The start time may correspond to a bedtime, and the end time may correspond to a wake-up time. The sleep mode schedule may be defined by designating a day of the week or weekdays/weekends. In addition, the sleep mode schedule may be defined by designating a date. In addition, the sleep mode schedule may be set to repeat in a predetermined pattern. In addition, the sleep mode schedule may be temporarily set for a particular date.

In addition, according to an embodiment of the present disclosure, the sleep mode schedule may be defined by designating a room or an area within the home. When the sleep mode schedule is defined by designating a room or an area within the home, the air conditioner 100 may obtain a sleep mode schedule for the room or area in which the air conditioner 100 is installed.

When the user completely inputs the sleep mode schedule, sleep mode schedule information 530 is generated and transmitted to the server 320. The server 320 may transmit the sleep mode schedule information 530 to the air conditioner 100.

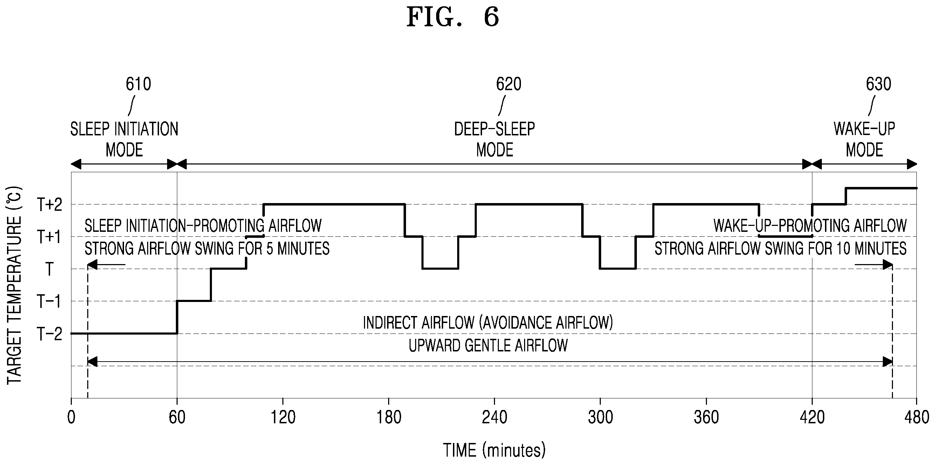

FIG. 6 is a diagram illustrating an operation of an air conditioning module in a sleep mode, according to an embodiment of the present disclosure.

In the graph of FIG. 6, the horizontal axis represents the elapsed time from a bedtime in minutes, and the vertical axis represents a target temperature corresponding to a set temperature of the air conditioning module 212. In the graph of FIG. 6, T represents a user-set temperature that is a temperature set by the user.

According to an embodiment of the present disclosure, in the sleep mode, the air conditioner 100 may adjust the set temperature of the air conditioning module 212 according to a sleep time. In addition, in the sleep mode, the air conditioner 100 may control at least one of an airflow direction, whether to perform an airflow swing, airflow intensity, whether to blow an indirect airflow/direct airflow, or whether to perform a windless operation, according to the sleep time of the air conditioning module 212.

According to an embodiment of the present disclosure, the sleep mode may include a sleep initiation mode 610, a deep-sleep mode 620, and a wake-up mode 630. The sleep initiation mode 610, the deep-sleep mode 620, and the wake-up mode 630 may be defined as predetermined time intervals. According to an embodiment of the present disclosure, the sleep initiation mode 610 may be defined as a period of 60 minutes from the bedtime, the deep-sleep mode 620 may be defined as a period from 60 minutes to 420 minutes from the bedtime, and the wake-up mode 630 may be defined as a period from 420 minutes to 480 minutes from the bedtime.

In the sleep initiation mode 610, the target temperature may be set lower than the user-set temperature T. For example, in the sleep initiation mode 610, the target temperature may be set 2 °C lower than the user-set temperature. In addition, in the sleep initiation mode 610, the airflow direction may be set to a sleep initiation-promoting airflow. The sleep initiation-promoting airflow may be set to, for example, maintain a strong swing airflow to output a strong airflow intermittently for 5 minutes. In the sleep initiation mode 610, rapid sleep initiation may be promoted through rapid cooling and a sleep initiation airflow. In the sleep initiation mode 610, the air conditioner 100 may induce deep sleep through sustained low-speed operation without perception of airflow. In addition, in the sleep initiation mode, the air conditioner 100 may perform an air purification function to prevent the user from catching a cold even at a low temperature.

When entering the deep-sleep mode 620 from the sleep initiation mode 610, the air conditioner 100 increases the target temperature to be higher than the user-set temperature T. For example, when entering the deep-sleep mode 620, the air conditioner 100 increases the target temperature to be 2 °C higher than the user-set temperature T. In the deep-sleep mode 620, the air conditioner 100 may maintain the target temperature to be higher than the user-set temperature T, while intermittently decreasing the target temperature to the user-set temperature T. For example, in the deep-sleep mode 620, the air conditioner 100 may, while maintaining the target temperature to be 2 °C higher than the user-set temperature T, decrease the target temperature to the user-set temperature T every hour. In the deep-sleep mode 620, the air conditioner 100 may operate with an indirect airflow, set the airflow intensity to be gentle, and set the airflow direction to be upward. In the deep-sleep mode 620, the air conditioner 100 may maintain a healthy-skin temperature. In addition, in the deep-sleep mode 620, the air conditioner 100 may increase the temperature to reduce the power consumption. In addition, in the deep-sleep mode 620, the air conditioner 100 may minimize a sensation of airflow at a sleeping position by using an avoidance airflow, and perform air stratification airflow control. In addition, in the deep-sleep mode 620, the air conditioner 100 may control the temperature in a wave-like pattern to ensure that the user remains asleep without waking and to secure rapid eye movement (REM) sleep.

In the wake-up mode 630, the air conditioner 100 may set the target temperature to be higher than the user-set temperature T. For example, in the wake-up mode 630, the air conditioner 100 may set the target temperature to be at least 2 °C higher than the user-set temperature T. In addition, in the wake-up mode 630, the air conditioner 100 may generate a wake-up-promoting airflow. The wake-up-promoting airflow may be, for example, an airflow in which a strong airflow is intermittently output for 10 minutes. In the wake-up mode 630, the air conditioner 100 controls the target temperature to correspond to a metabolism-activating temperature and allows the user to refreshingly wake up through airflow stimulation.

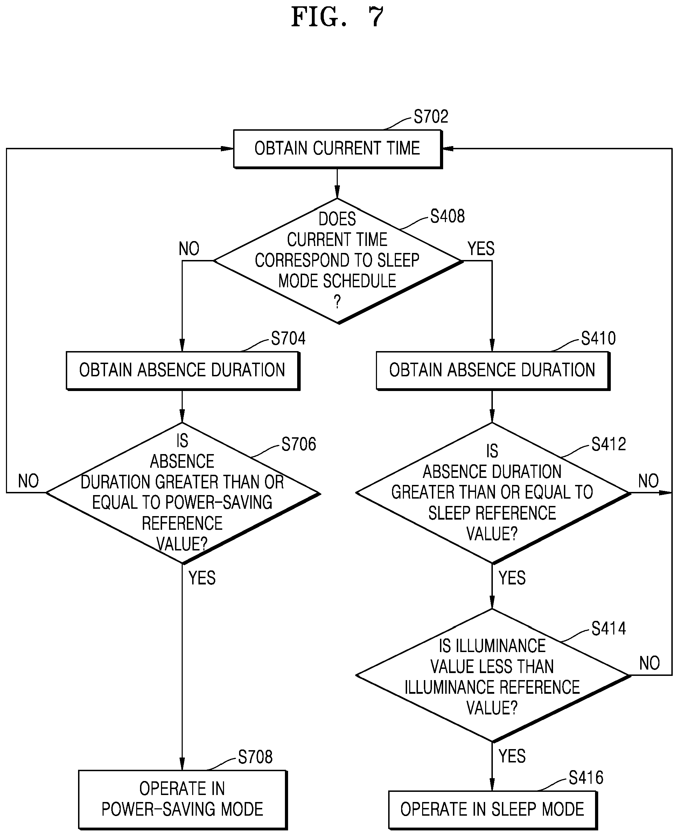

FIG. 7 is a diagram illustrating a method of controlling an air conditioner, according to an embodiment of the present disclosure.

According to an embodiment of the present disclosure, the air conditioner 100 may, when the current time corresponds to the sleep mode, determine whether to operate in the sleep mode based on an absence duration, and when the current time does not correspond to the sleep mode, determine whether to operate in a power-saving mode based on the absence duration.

Referring to FIG. 7, in operation S702, the air conditioner 100 obtains the current time. Next, in operation S408, the air conditioner 100 determines whether the current time corresponds to a sleep mode schedule.

When the current time corresponds to the sleep mode schedule, the air conditioner 100 performs operations S410, S412, S414, and S416. Operations S410, S412, S414, and S416 are the same as those described above with reference to FIG. 4, and thus, detailed descriptions thereof will be omitted.

When the current time does not correspond to the sleep mode schedule, in operation S704, the air conditioner 100 obtains an absence duration. In the same manner as in operation S410 described above, the air conditioner 100 may detect a motion value and obtain the absence duration, based on a sensor detection value of the detection sensor 110.

Next, in operation S706, the air conditioner 100 determines whether the absence duration is greater than or equal to a power-saving reference value. According to an embodiment of the present disclosure, the power-saving reference value may be the same value as the sleep reference value. In addition, according to an embodiment of the present disclosure, the power-saving reference value may be different from the sleep reference value.

When the absence duration is greater than or equal to the power-saving reference value, in operation S708, the air conditioner 100 may operate in a power-saving mode. The power-saving mode is a mode for increasing the set temperature of the air conditioner 100 or turning off the air conditioner 100. According to an embodiment of the present disclosure, in the power-saving mode, the air conditioner 100 may be powered off while keeping the detection sensor 110 activated in a soft-off state. When motion of a user is detected by the detection sensor 110 after entering the power-saving mode, the air conditioner 100 may terminate the power-saving mode and perform an air conditioning operation with the previous set temperature.

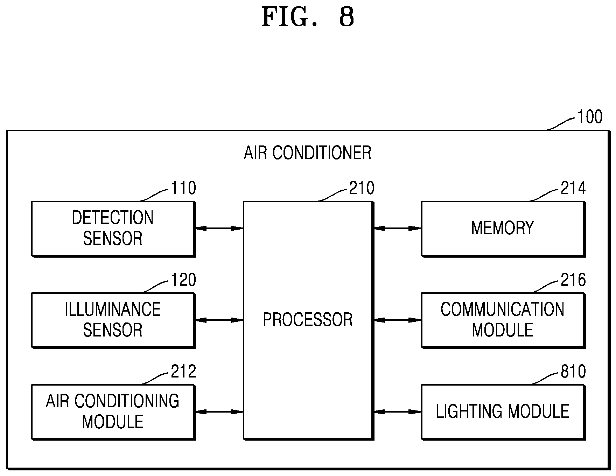

FIG. 8 is a diagram illustrating a structure of an air conditioner according to an embodiment of the present disclosure.

According to an embodiment of the present disclosure, the air conditioner 100 includes the detection sensor 110, the illuminance sensor 120, the processor 210, the air conditioning module 212, the memory 214, the communication module 216, and a lighting module 810. The block diagram of the air conditioner 100 of FIG. 8 may correspond to a block diagram of an indoor unit. The air conditioner 100 of FIG. 8 will be described focusing on differences from the air conditioner 100 of FIG. 2. Descriptions of the detection sensor 110, the illuminance sensor 120, the processor 210, the air conditioning module 212, the memory 214, and the communication module 216 of the air conditioner 100 of FIG. 8, which are redundant to those provided above with reference to FIG. 2, will be omitted.

The air conditioner 100 may include the lighting module 810. The lighting module 810 includes a light-emitting element that outputs light. The lighting module 810 may be arranged to face the target space 160 to output light toward the target space 160. The lighting module 810 may include, for example, an LED.

The lighting module 810 may be turned on or off, or its luminous intensity may be adjusted, by a user input through an input interface or a user input received from the external device 310. In addition, according to an embodiment of the present disclosure, when operating in the sleep mode, the processor 210 may control the lighting module 810.

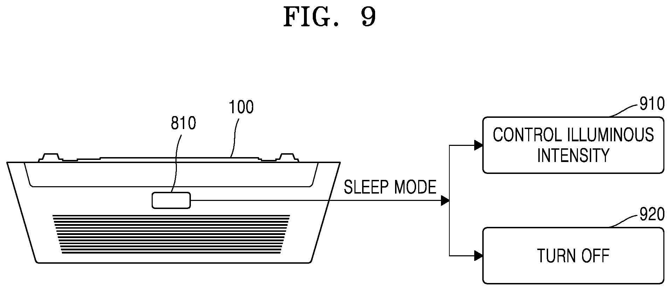

FIG. 9 is a diagram illustrating control of a lighting module in a sleep mode, according to an embodiment of the present disclosure.

According to an embodiment of the present disclosure, when operating in the sleep mode, the processor 210 may turn off the lighting module 810 or lower its luminous intensity.

According to an embodiment of the present disclosure, when operating in the sleep mode, in operation 910, the processor 210 controls the luminous intensity of the lighting module 810. In the sleep mode, the processor 210 may set the luminous intensity of the lighting module 810 to a low level corresponding to a predetermined sleep illuminance. The sleep illuminance may be set within a range of 50 to 100 lux.

In addition, according to an embodiment of the present disclosure, when operating in the sleep mode, in operation 920, the processor 210 turns off the lighting module 810.

According to an embodiment of the present disclosure, when operating in the sleep mode, the processor 210 may set the luminous intensity of the lighting module 810 to a low level corresponding to the sleep illuminance for a predetermined time period, and turn off the lighting module 810 after the predetermined time period.

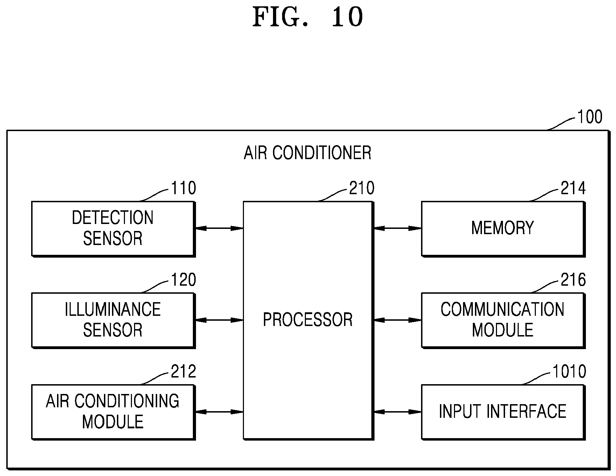

FIG. 10 is a diagram illustrating a structure of an air conditioner according to an embodiment of the present disclosure.

According to an embodiment of the present disclosure, the air conditioner 100 includes the detection sensor 110, the illuminance sensor 120, the processor 210, the air conditioning module 212, the memory 214, the communication module 216, and an input interface 1010. The block diagram of the air conditioner 100 of FIG. 10 may correspond to a block diagram of an indoor unit. The air conditioner 100 of FIG. 10 will be described focusing on differences from the air conditioner 100 of FIG. 2. Descriptions of the detection sensor 110, the illuminance sensor 120, the processor 210, the air conditioning module 212, the memory 214, and the communication module 216 of the air conditioner 100 of FIG. 10, which are redundant to those provided above with reference to FIG. 2, will be omitted.

According to an embodiment of the present disclosure, the air conditioner 100 includes the input interface 1010.

The input interface 1010 receives an input from a user. The input interface 1010 may include keys, a touch screen, a touch pad, a touch sensor, or the like. The input interface 1010 receives a user input and delivers it to the processor 210. The input interface 1010 may receive a power on/off signal, a temperature setting signal, an operation mode selection signal, an airflow intensity selection signal, a sleep scheduling signal, a scheduled operation setting signal, an airflow direction setting signal, or the like.

According to an embodiment of the present disclosure, a user input for setting the sleep mode may be received through the input interface 1010. When a user input for setting the sleep mode is received, the processor 210 operates in the sleep mode. When the sleep mode is initiated by the user input, the processor 210 may stop a process of determining whether to enter the sleep mode based on an absence duration and an illuminance value.

According to an embodiment of the present disclosure, when entering the sleep mode by a user input, the processor 210 may deactivate at least one of the detection sensor 110 or the illuminance sensor 120. In this case, the deactivated detection sensor 110 or illuminance sensor 120 may remain deactivated until the sleep mode is terminated, and may be activated again when the sleep mode is terminated.

In addition, according to an embodiment of the present disclosure, when entering the sleep mode by a user input, the processor 210 may activate at least one of the detection sensor 110 or the illuminance sensor 120 and operate. Even when at least one of the detection sensor 110 or the illuminance sensor 120 is activated to operate as described above, the processor 210 may stop the process of determining whether to enter the sleep mode based on an absence duration and an illuminance value.

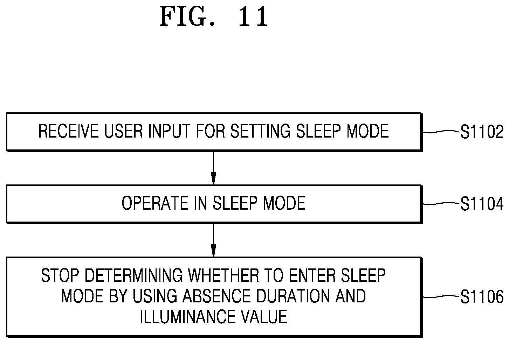

FIG. 11 is a diagram illustrating a method of controlling an air conditioner, according to an embodiment of the present disclosure.

When a user input for setting the sleep mode is input by a user, the air conditioner 100 may enter the sleep mode and operate.

Referring to FIG. 11, in operation S1102, the air conditioner 100 receives a user input for setting the sleep mode. The user input may be input through the input interface 1010 of the air conditioner 100 or may be input through the external device 310.

Next, in operation S1104, the air conditioner 100 operates in the sleep mode based on the user input. The air conditioner 100 may control the air conditioning module 212 or control the lighting module 810 based on the sleep mode.

Next, in operation S1106, by operating in the sleep mode, the air conditioner 100 stops the process of determining whether to enter the sleep mode based on an absence duration and an illuminance value.

According to an embodiment of the present disclosure, in operation S1106, the air conditioner 100 may deactivate at least one of the detection sensor 110 or the illuminance sensor 120. According to an embodiment of the present disclosure, in operation S1106, the air conditioner 100 may activate the detection sensor 110 and deactivate the illuminance sensor 120. In addition, according to an embodiment of the present disclosure, in operation S1106, the air conditioner 100 may deactivate both the detection sensor 110 and the illuminance sensor 120. In addition, according to an embodiment of the present disclosure, in operation S1106, the air conditioner 100 may activate both the detection sensor 110 and the illuminance sensor 120.

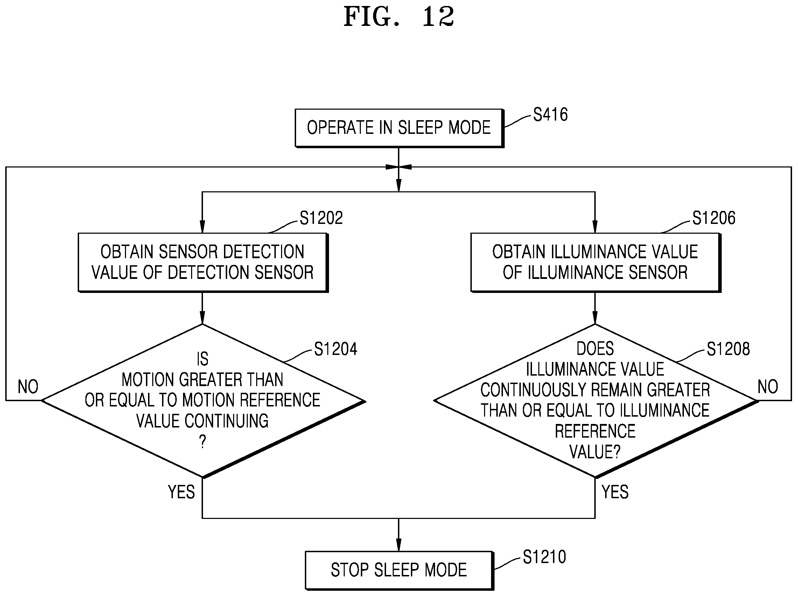

FIG. 12 is a view illustrating a process of stopping a sleep mode during a sleep mode operation, according to an embodiment of the present disclosure.

According to an embodiment of the present disclosure, while operating in the sleep mode, the air conditioner 100 may stop the sleep mode based on a sensor detection value of the detection sensor 110 or the illuminance sensor 120.

Referring to FIG. 12, while operating in the sleep mode in operation S416, the air conditioner 100 may obtain a sensor detection value of the detection sensor 110 in operation S1202. In operation S1204, based on the sensor detection value of the detection sensor 100, the air conditioner 100 may determine whether a motion value continuously remains greater than or equal to a motion reference value. The air conditioner 100 may determine whether the motion value continuously remains greater than or equal to the motion reference value for a first reference time period. When the motion value continuously remains greater than or equal to the motion reference value for the first reference time period, in operation S1210, the air conditioner 100 may stop the sleep mode.

While operating in the sleep mode in operation S416, the air conditioner 100 may obtain an illuminance value of the illuminance sensor 120 in operation S1206. In operation S1208, the air conditioner 100 may determine whether the illuminance value continuously remains greater than or equal to an illuminance reference value. When the illuminance value continuously remains greater than or equal to the illuminance reference value for a second reference time period, in operation S1210, the air conditioner 100 may stop the sleep mode.

According to an embodiment of the present disclosure, the first reference time period and the second reference time period may be equal to each other. In addition, according to an embodiment of the present disclosure, the first reference time period and the second reference time period may be different from each other. According to an embodiment of the present disclosure, the first reference time period and the second reference time period may be less than or equal to the sleep reference value.

When the sleep mode is stopped in operation S1210, the air conditioner 100 may control the air conditioning module 212 to operate with the previous user-set temperature.

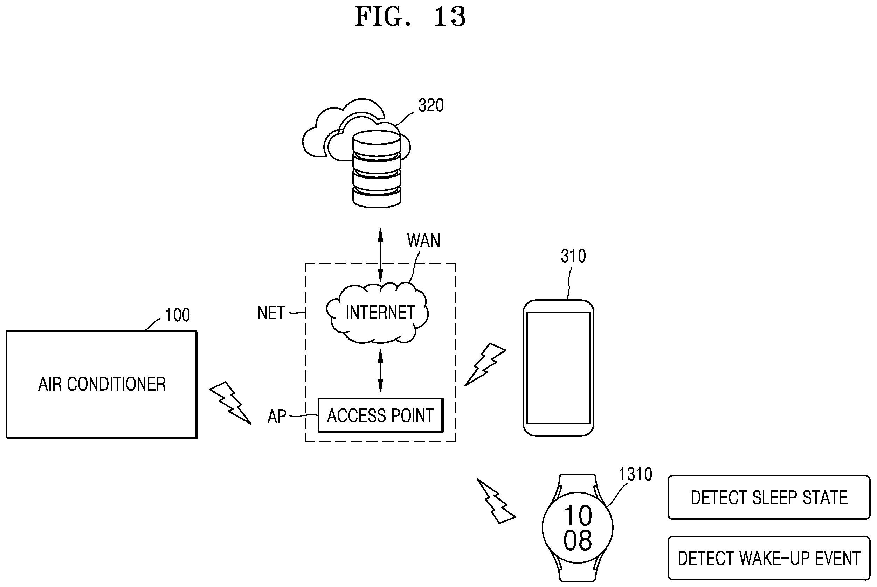

FIG. 13 is a diagram illustrating an air conditioner, an external device, a wearable device, and a server, according to an embodiment of the present disclosure.

According to an embodiment of the present disclosure, the air conditioner 100 communicates with the external device 310 and the server 320 via the communication module 216. The air conditioner 100 may be connected to other home appliances, the external device 310, or the server 320, via the network NET.

According to an embodiment of the present disclosure, the air conditioner 100 may communicate with a wearable device 1310 via the network NET. The wearable device 1310 may correspond to, for example, a watch or glasses. According to an embodiment of the present disclosure, the wearable device 1310 may be connected to the external device 310 in the form of a smart phone or tablet personal computer (PC), through short-range communication, and may communicate with the air conditioner 100 via the external device 310 and the server 320. In addition, according to an embodiment of the present disclosure, the wearable device 1310 may be connected to the server 320 through mobile communication, and may communicate with the air conditioner 100 via the server 320.

According to an embodiment of the present disclosure, the wearable device 1310 may include a motion sensor configured to detect a motion of the user. The wearable device 1310 may detect a sleep state of the user, or a wake-up event in which the user wakes up, based on a motion value.

In addition, according to an embodiment of the present disclosure, the wearable device 1310 may include a biosensor. The biosensor may include, for example, a heart rate sensor, a body temperature sensor, a blood pressure sensor, an oxygen saturation sensor, an electrocardiogram sensor, or the like. The wearable device 1310 may detect a sleep state of the user or a wake-up event by using a sensor detection value of the biosensor.

The wearable device 1310 may detect a sleep state of the user or a wake-up event by using sensor detection values of the motion sensor and the biosensor together.

The wearable device 1310 may transmit, to the server 320, information about the detected sleep state of the user or the detected wake-up event. The server 320 may transmit, to the air conditioner 100, sleep state information or wake-up event information received from the wearable device 1310. The air conditioner 100 may control the sleep mode based on the sleep state information or the wake-up event information received from the wearable device 1310.

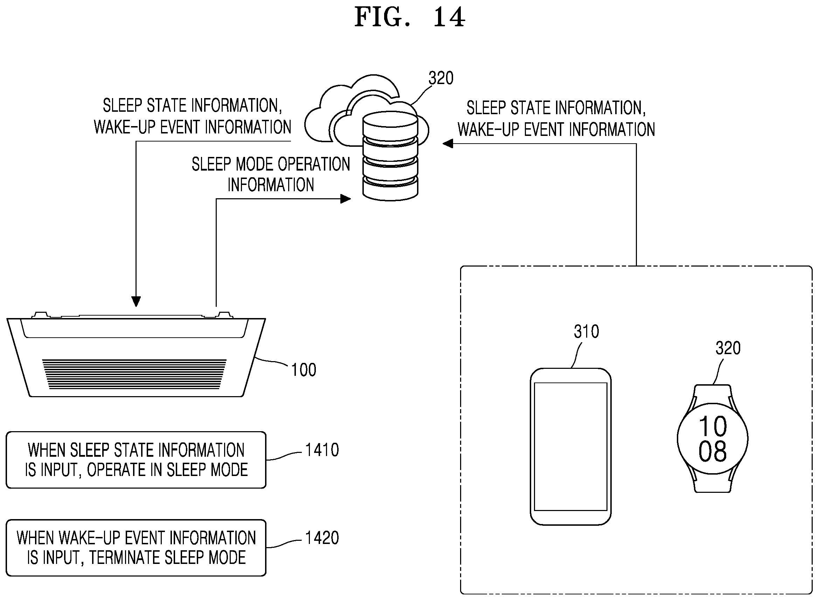

FIG. 14 is a diagram illustrating an operation of controlling a sleep mode of an air conditioner, according to an embodiment of the present disclosure.

According to an embodiment of the present disclosure, the air conditioner 100 may receive sleep state information or wake-up event information from the external device 310 or the wearable device 1310. For example, in a case where the external device 310 corresponds to a smart phone, the external device 310 may detect a sleep state of the user or a wake-up event by using alarm information. In addition, when the user sets the sleep mode, the external device 310 may recognize that the user is in a sleep state. In addition, when the user manipulates the external device 310 operating in the sleep mode, the external device 310 may detect a wake-up event of the user. As described above, the wearable device 1310 may detect a sleep state of the user or a wake-up event by using a sensor detection value of the motion sensor or the biosensor. According to an embodiment of the present disclosure, the external device 310 or the wearable device 1310 may transmit sleep state information or wake-up event information to the server 320.

According to an embodiment of the present disclosure, when operating in the sleep mode, the air conditioner 100 transmits, to the server 320, sleep mode operation information indicating that the air conditioner 100 is operating in the sleep mode. The server 320 may obtain the information that the air conditioner 100 is operating in the sleep mode. In addition, the server 320 may monitor the air conditioner 100 and obtain the sleep mode operation information.

When sleep state information or wake-up event information is received from the external device 310 or the wearable device 1310, the server 320 transmits the received information to the air conditioner 100.

According to an embodiment of the present disclosure, the server 320 may determine whether to transmit sleep state information or wake-up event information to the air conditioner 100, according to whether the air conditioner 100 is operating in the sleep mode. According to an embodiment of the present disclosure, when sleep state information is received while the air conditioner 100 is operating in the sleep mode, the server 320 may not transmit the sleep state information to the air conditioner 100. In addition, when wake-up event information is received while the air conditioner 100 is operating in the sleep mode, the server 320 may transmit the wake-up event information to the air conditioner 100. In addition, when sleep state information is received while the air conditioner 100 is not operating in the sleep mode, the server 320 may transmit the sleep state information to the air conditioner 100. In addition, when wake-up event information is received while the air conditioner 100 is not operating in the sleep mode, the server 320 may transmit the wake-up event information to the air conditioner 100.

According to an embodiment of the present disclosure, when sleep state information is received from the server 320 while the air conditioner 100 is not operating in the sleep mode, in operation 1410, the air conditioner 100 may operate in the sleep mode. According to an embodiment of the present disclosure, when the current time corresponds to a sleep mode schedule, and sleep state information is received from the server 320, the air conditioner 100 may operate in the sleep mode. When the sleep state information is received from the server 320 but the current time does not correspond to the sleep mode schedule, the air conditioner 100 may not operate in the sleep mode.

In addition, according to an embodiment of the present disclosure, when wake-up event information is received from the server 320 while the air conditioner 100 is operating in the sleep mode, in operation 1420, the air conditioner 100 may terminate the sleep mode.

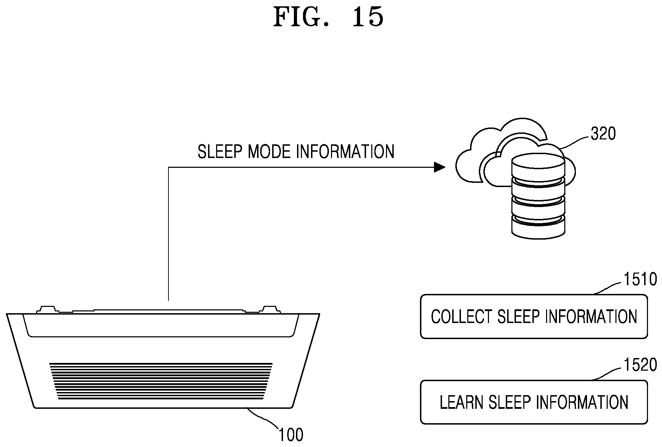

FIG. 15 is a diagram illustrating an operation of a server according to sleep mode information of an air conditioner, according to an embodiment of the present disclosure.

According to an embodiment of the present disclosure, when operating in the sleep mode, the air conditioner 100 transmits, to the server 320, sleep mode information indicating that the air conditioner 100 is operating in the sleep mode. The air conditioner 100 may transmit sleep mode start time information along with the sleep mode information.

According to an embodiment of the present disclosure, in operation 1510, the server 320 may collect sleep information based on the sleep mode information received from the air conditioner 100. The sleep information may include, for example, bedtime information, sleep duration information, wake-up time information, or the like.

In operation 1520, the server 320 may learn sleep information of the user based on the collected sleep information. According to an embodiment of the present disclosure, the server 320 may generate sleep schedule information by using the learned sleep information, and transmit the sleep schedule information to the air conditioner 100.

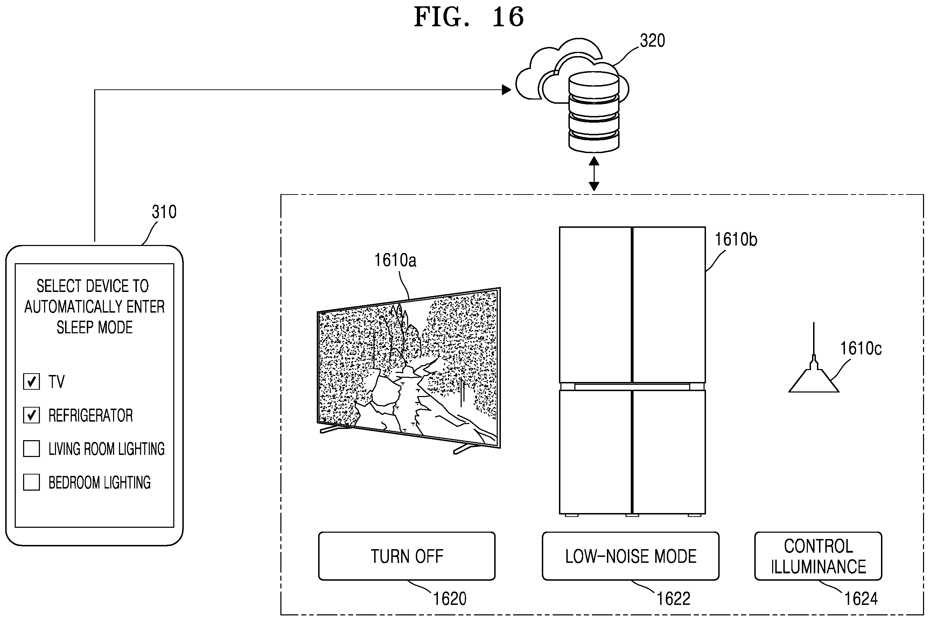

FIG. 16 is a diagram illustrating a process of controlling a home appliance based on sleep mode information of an air conditioner, according to an embodiment of the present disclosure.

According to an embodiment of the present disclosure, when sleep mode information is received from the air conditioner 100, the server 320 may control another home appliance 1610a, 1610b, or 1610c to operate in a sleep mode.

The home appliance 1610a, 1610b, or 1610c to operate in a sleep mode when the air conditioner 100 is in the sleep mode may be selected in advance by the user. According to an embodiment of the present disclosure, the external device 310 may provide a GUI for selecting the home appliance 1610a, 1610b, or 1610c to operate in a sleep mode based on sleep mode information of the air conditioner 100. The server 320 may receive selection information of the home appliance 1610a, 1610b, or 1610c from the external device 310.

According to an embodiment of the present disclosure, when sleep mode information is received from the air conditioner 100, the server 320 may control the selected home appliance 1610a, 1610b, or 1610c to operate in the sleep mode. For example, in a case where the home appliance 1610a is a television (TV), in operation 1620, the server 320 may turn off the home appliance 1610a, based on the sleep mode information of the air conditioner 100. In the sleep mode, when the TV is playing video content or audio content, the server 320 may control the TV to stop playing the video content or playing the audio content. In addition, for example, in a case where the home appliance 1610b is a refrigerator, in operation 1622, the server 320 may control the home appliance 1610b to operate in a low-noise mode, based on the sleep mode information of the air conditioner 100. In addition, for example, in a case where the home appliance 1610c is a lighting device, in operation 1624, the server 320 may turn off the home appliance 1610c or lower its luminous intensity to correspond to a sleep illuminance, based on the sleep mode information of the air conditioner 100.

According to an embodiment of the present disclosure, there is an effect of providing a better sleep environment to a user by controlling another home appliance 1610a, 1610b, or 1610c in the home to operate in a sleep mode based on the sleep mode information of the air conditioner 100.

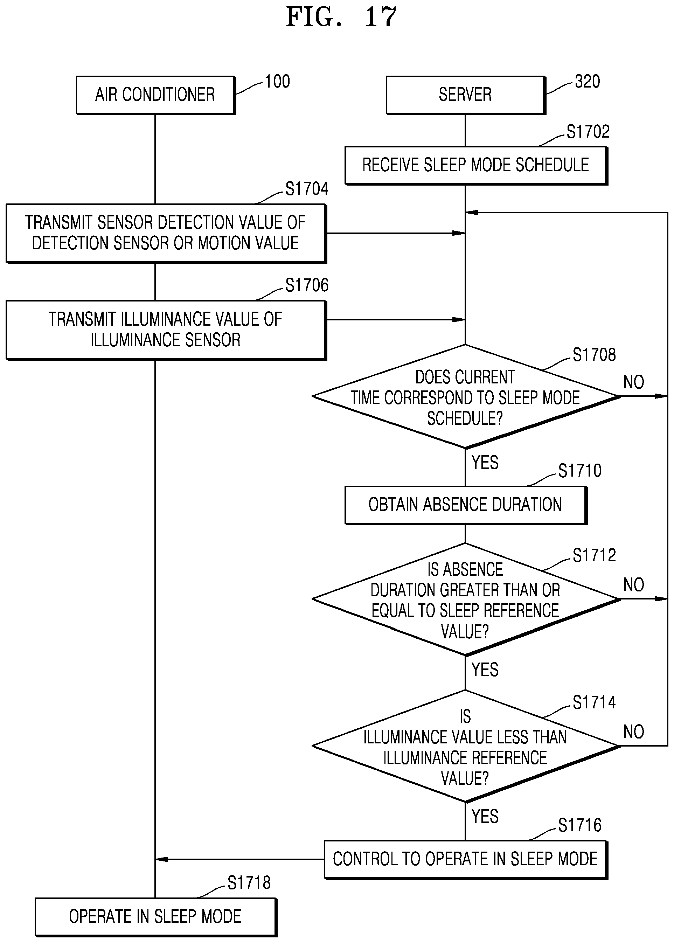

FIG. 17 is a diagram illustrating a method of controlling an air conditioner, according to an embodiment of the present disclosure.

According to an embodiment of the present disclosure, operations of the method of controlling an air conditioner may be performed by the server 320.

In operation S1702, the server 320 may receive sleep mode schedule information. The server 320 may receive sleep mode schedule information input through an application of the external device 310.

Next, in operation S1704, according to an embodiment of the present disclosure, the air conditioner 100 may transmit a sensor detection value of the detection sensor 110 to the server 320. The server 320 may calculate a motion value based on the sensor detection value of the detection sensor 110.

In addition, according to an embodiment of the present disclosure, in operation S1704, the air conditioner 100 may calculate the motion value by using the sensor detection value of the detection sensor 110, and transmit the calculated motion value to the server 320. In this case, the motion value calculation process may not be performed by the server 320.

Next, in operation S1706, the air conditioner 100 may transmit an illuminance value of the illuminance sensor to the server 320.

The order of operation S1704 and operation S1706 is not limited to the order illustrated in FIG. 17, and S1704 and S1706 may be performed according to various orders.

Next, in operation S1708, the server 320 determines whether the current time corresponds to a sleep mode schedule. When the current time corresponds to the sleep mode schedule, in operation S1710, the server 320 obtains an absence duration. The server 320 may calculate the absence duration by using the sensor detection value of the detection sensor 110 or the motion value received from the air conditioner 100. According to an embodiment of the present disclosure, the air conditioner 100 may calculate the absence duration and transmit the calculated absence duration to the server 320.

Next, in operation S1712, the server 320 may determine whether the absence duration is greater than or equal to a sleep reference value. When the absence duration is greater than or equal to the sleep reference value, in operation S1714, the server 320 may determine whether the illuminance value is less than an illuminance reference value.

When the absence duration is greater than or equal to the sleep reference value, and the illuminance value is less than the illuminance reference value, in operation S1716, the server 320 controls the air conditioner 100 to operate in the sleep mode. In operation S1718, the air conditioner 100 may operate in the sleep mode under control of the server 320.

A machine-readable storage medium may be provided in the form of a non-transitory storage medium. Here, the term 'non-transitory storage medium' refers to a tangible device and does not include a signal (e.g., an electromagnetic wave), and the term 'non-transitory storage medium' does not distinguish between a case where data is stored in a storage medium semi-permanently and a case where data is stored temporarily. For example, the 'non-transitory storage medium' may include a buffer in which data is temporarily stored.

According to an embodiment, methods according to various embodiments disclosed herein may be included in a computer program product and then provided. The computer program product may be traded as a commodity between sellers and buyers. The computer program product may be distributed in the form of a machine-readable storage medium (e.g., a compact disc ROM (CD-ROM)), or may be distributed online (e.g., downloaded or uploaded) through an application store or directly between two user devices (e.g., smart phones). In a case of online distribution, at least a portion of the computer program product (e.g., a downloadable app) may be temporarily stored in a machine-readable storage medium such as a manufacturer's server, an application store's server, or memory of a relay server.

According to an aspect of an embodiment of the present disclosure, an air conditioner 100 is provided. The air conditioner 100 may include a detection sensor 110 configured to detect an object, an illuminance sensor 120 configured to detect illuminance, an air conditioning module 212, a communication module 216, memory 214 storing at least one instruction, and at least one processor 210. The at least one processor 210 may execute the at least one instruction to receive, from an external device 310 through the communication module 216, a sleep mode schedule input by a user, obtain, while a current time corresponds to the sleep mode schedule, an absence duration during which a person is not detected in a target space, based on a sensor detection value of the detection sensor 110, and control, based on the absence duration being greater than or equal to a sleep reference value and based on an illuminance value detected by the illuminance sensor 120 being less than an illuminance reference value, the air conditioning module to operate in a sleep mode.

In addition, according to an embodiment of the present disclosure, the at least one processor 210 may execute the at least one instruction to adjust a target temperature in a preset pattern while operating in the sleep mode.

In addition, according to an embodiment of the present disclosure, the sleep mode may include a sleep initiation mode in which the target temperature is set lower than a user-set temperature that is set by the user, a deep-sleep mode in which the target temperature is adjusted according to a sleep pattern, and a wake-up mode in which the target temperature is set higher than the user-set temperature.

In addition, according to an embodiment of the present disclosure, the at least one processor 210 may execute the at least one instruction to, based on the current time not corresponding to the sleep mode schedule, obtain the absence duration based on the sensor detection value of the detection sensor 110, and based on the absence duration being greater than or equal to a power-saving reference value, operate in a power saving mode.

In addition, according to an embodiment of the present disclosure, the at least one processor 210 may execute the at least one instruction to detect a motion value of an object in the target space based on the sensor detection value of the detection sensor, and calculate the absence duration by counting a duration during which the motion value of the object in the target space remains less than a motion reference value.

In addition, according to an embodiment of the present disclosure, the air conditioner 100 may further include a lighting module 810 configured to emit light toward the target space, and the at least one processor 210 may execute the at least one instruction to, based on operating in the sleep mode, perform at least one of lowering a luminous intensity of the lighting module 810 to correspond to a sleep illuminance or turning off the lighting module 810.

In addition, according to an embodiment of the present disclosure, the air conditioner 100 may further include an input interface 1010, and the at least one processor 210 may execute the at least one instruction to, based on receiving, through the input interface 1010 or the communication module 216, a user input for requesting to operate in the sleep mode, control the air conditioning module 212 to operate in the sleep mode, and stop determining, based on the absence duration and the illuminance value, whether to operate in the sleep mode.

In addition, according to an embodiment of the present disclosure, the at least one processor 210 may execute the at least one instruction to, based on a motion greater than or equal to the motion reference value continuing for a first reference time period based on the sensor detection value of the detection sensor 110 while operating in the sleep mode, stop the sleep mode, and based on the illuminance value detected by the illuminance sensor 120 remaining greater than or equal to the illuminance reference value for a second reference time period while operating in the sleep mode, stop the sleep mode.

In addition, according to an embodiment of the present disclosure, the at least one processor 210 may execute the at least one instruction to, based on operating in the sleep mode, deactivate the detection sensor and the illuminance sensor 120.

In addition, according to an embodiment of the present disclosure, the at least one processor 210 may execute the at least one instruction to, based on receiving, from a wearable device 1310 worn by the user through the communication module 216, sleep state information indicating that the user is in a sleep state, control the air conditioning module 212 to operate in the sleep mode.

In addition, according to an embodiment of the present disclosure, the at least one processor 210 may execute the at least one instruction to, based on receiving wake-up event information of the user from the external device 310 through the communication module 216 while operating in the sleep mode, terminate the sleep mode.

In addition, according to an embodiment of the present disclosure, the at least one processor 210 may execute the at least one instruction to, based on operating in the sleep mode, transmit, to a server 320 through the communication module 216, sleep mode information indicating that the air conditioner 100 is operating in the sleep mode.

In addition, according to an embodiment of the present disclosure, the sleep mode schedule may include at least one of bedtime information, sleep duration information, day-of-the-week information, or date information.

In addition, according to an aspect of an embodiment of the present disclosure, a method of controlling an air conditioner is provided. The method of controlling an air conditioner may include receiving a sleep mode schedule input by a user, obtaining a sensor detection value of a detection sensor of the air conditioner in a home, obtaining an illuminance value detected by an illuminance sensor of the air conditioner, obtaining, while a current time corresponds to the sleep mode schedule, an absence duration during which a person is not detected in a target space, based on the sensor detection value of the detection sensor, and based on the absence duration being greater than or equal to a sleep reference value and based on the illuminance value being less than an illuminance reference value, controlling the air conditioner to operate in a sleep mode.

In addition, according to an embodiment of the present disclosure, the method of controlling an air conditioner may further include controlling at least one home appliance in a home to operate in a sleep mode, based on information that the air conditioner is operating in the sleep mode.

In addition, according to an embodiment of the present disclosure, the method of controlling an air conditioner may further include receiving a user input for selecting at least one home appliance to operate in the sleep mode based on the air conditioner operating in the sleep mode, and the controlling of the at least one home appliance in the home to operate in the sleep mode may include controlling the at least one home appliance selected by the user input to operate in the sleep mode.

In addition, according to an embodiment of the present disclosure, the controlling of the at least one home appliance in the home to operate in the sleep mode may include at least one of controlling a TV in the home to stop playing video content or audio content, controlling the at least one home appliance in the home to operate in a low-noise mode, and performing control such that a luminous intensity of a lighting device in the home is lowered to correspond to a sleep illuminance or the lighting device is turned off.

In addition, according to an embodiment of the present disclosure, the method of controlling an air conditioner may further include collecting sleep information of the user based on information that the air conditioner is operating in the sleep mode.

In addition, according to an embodiment of the present disclosure, the obtaining of the sensor detection value of the detection sensor may include receiving the sensor detection value of the detection sensor or a motion value from the air conditioner, the obtaining of the illuminance value of the illuminance sensor may include receiving the illuminance value from the air conditioner, and the controlling of the air conditioner to operate in the sleep mode may include transmitting a control signal for operating in the sleep mode to the air conditioner.

In addition, according to an aspect of an embodiment of the present disclosure, provided is a computer-readable recording medium having recorded thereon a program for causing a computer to execute the method of controlling an air conditioner.

## Claims

1. An air conditioner (100) comprising:
a detection sensor (110) configured to detect an object;
an illuminance sensor (120) configured to detect illuminance;
an air conditioning module (212);
a communication module (216);
memory (214) storing at least one instruction; and
at least one processor (210) configured to execute the at least one instruction to receive, from an external device (310) through the communication module (216), a sleep mode schedule input by a user, obtain, while a current time corresponds to the sleep mode schedule, an absence duration during which a person is not detected in a target space, based on a sensor detection value of the detection sensor (110), and control, based on the absence duration being greater than or equal to a sleep reference value and based on an illuminance value detected by the illuminance sensor (120) being less than an illuminance reference value, the air conditioning module to operate in a sleep mode.

2. The air conditioner (100) of claim 1, wherein the at least one processor (210) is further configured to execute the at least one instruction to adjust a target temperature in a preset pattern while operating in the sleep mode.

3. The air conditioner (100) of claim 1 or 2, wherein the sleep mode includes a sleep initiation mode in which the target temperature is set lower than a user-set temperature that is set by the user, a deep-sleep mode in which the target temperature is adjusted according to a sleep pattern, and a wake-up mode in which the target temperature is set higher than the user-set temperature.

4. The air conditioner (100) of any one of claims 1 to 3, wherein the at least one processor (210) is further configured to execute the at least one instruction to, based on the current time not corresponding to the sleep mode schedule, obtain the absence duration based on the sensor detection value of the detection sensor (110), and based on the absence duration being greater than or equal to a power-saving reference value, operate in a power saving mode.

5. The air conditioner (100) of any one of claims 1 to 4, wherein the at least one processor (210) is further configured to execute the at least one instruction to detect a motion value of an object in the target space based on the sensor detection value of the detection sensor, and calculate the absence duration by counting a duration during which the motion value of the object in the target space remains less than a motion reference value.

6. The air conditioner (100) of any one of claims 1 to 5, further comprising a lighting module (810) configured to emit light toward the target space, wherein the at least one processor (210) is further configured to execute the at least one instruction to, based on operating in the sleep mode, perform at least one of lowering a luminous intensity of the lighting module (810) to correspond to a sleep illuminance or turning off the lighting module (810).

7. The air conditioner (100) of any one of claims 1 to 6, further comprising an input interface (1010),
wherein the at least one processor (210) is further configured to execute the at least one instruction to, based on receiving, through the input interface (1010) or the communication module (216), a user input for requesting to operate in the sleep mode, control the air conditioning module (212) to operate in the sleep mode, and stop determining, based on the absence duration and the illuminance value, whether to operate in the sleep mode.

8. The air conditioner (100) of any one of claims 1 to 7, wherein the at least one processor (210) is further configured to execute the at least one instruction to, based on a motion greater than or equal to the motion reference value continuing for a first reference time period based on the sensor detection value of the detection sensor (110) while operating in the sleep mode, stop the sleep mode, and based on the illuminance value detected by the illuminance sensor (120) remaining greater than or equal to the illuminance reference value for a second reference time period while operating in the sleep mode, stop the sleep mode.

9. The air conditioner (100) of any one of claims 1 to 8, wherein the at least one processor (210) is further configured to execute the at least one instruction to, based on operating in the sleep mode, deactivate the detection sensor and the illuminance sensor (120).

10. The air conditioner (100) of any one of claims 1 to 9, wherein the at least one processor (210) is further configured to execute the at least one instruction to, based on receiving, from a wearable device (1310) worn by the user through the communication module (216), sleep state information indicating that the user is in a sleep state, control the air conditioning module (212) to operate in the sleep mode.

11. The air conditioner (100) of any one of claims 1 to 10, wherein the at least one processor (210) is further configured to execute the at least one instruction to, based on receiving wake-up event information of the user from the external device (310) through the communication module (216) while operating in the sleep mode, terminate the sleep mode.

12. The air conditioner (100) of any one of claims 1 to 11, wherein the at least one processor (210) is further configured to execute the at least one instruction to, based on operating in the sleep mode, transmit, to a server (320) through the communication module (216), sleep mode information indicating that the air conditioner (100) is operating in the sleep mode.

13. The air conditioner (100) of any one of claims 1 to 12, wherein the sleep mode schedule includes at least one of bedtime information, sleep duration information, day-of-the-week information, or date information.

14. A method of controlling an air conditioner, the method comprising:
receiving a sleep mode schedule input by a user;
obtaining a sensor detection value of a detection sensor of the air conditioner in a home;
obtaining an illuminance value detected by an illuminance sensor of the air conditioner;
obtaining, while a current time corresponds to the sleep mode schedule, an absence duration during which a person is not detected in a target space, based on the sensor detection value of the detection sensor; and
based on the absence duration being greater than or equal to a sleep reference value and based on the illuminance value being less than an illuminance reference value, controlling the air conditioner to operate in a sleep mode.

15. A computer-readable recording medium having recorded thereon a program for causing a computer to execute the method of claim 14.
